# EUROPEAN PATENT APPLICATION

(11) **EP 4 206 224 A1**
(43) Date of publication of application: **05.07.2023**
(21) Application number: 21861003.8
(22) Date of filing: 09.07.2021
(51) Int. Cl.: C07K 16/10, A61K 39/215, C12N 15/13, C12P 21/08

(54) **HUMAN ANTIBODY OR ANTIGEN-BINDING FRAGMENT THEREOF AGAINST CORONAVIRUS SPIKE PROTEIN**

(30) Priority: 26.08.2020 JP 2020143055; 26.03.2021 JP 2021054387
(71) Applicant: National University Corporation Kumamoto University, Kumamoto-shi, Kumamoto 860-8555 (JP); The University of Tokyo, Bunkyo-ku, Tokyo 113-8654 (JP)
(72) Inventor: MATSUSHITA, Shuzo, Kumamoto-shi Kumamoto 860-8555 (JP); KAKU, Yu, Kumamoto-shi Kumamoto 860-8555 (JP); KUWATA, Takeo, Kumamoto-shi Kumamoto 860-8555 (JP); KAWAOKA, Yoshihiro, Tokyo 113-8654 (JP); IMAI, Masaki, Tokyo 113-8654 (JP); YAMAYOSHI, Seiya, Tokyo 113-8654 (JP)
(74) Representative: Maiwald GmbH
(86) International application number: PCT/JP2021/026053
(87) International publication number: WO 2022/044573

(57) **Abstract**

It is an object of the present invention to provide antibodies against coronavirus (SARS-CoV-2). It is also an object of the present invention to provide a pharmaceutical composition against coronavirus infection using the antibody. According to the present invention, an antibody or antigen-binding fragment thereof that binds to the receptor-binding domain present in the spike protein of coronavirus and is capable of neutralizing the coronavirus, and a pharmaceutical composition for the prevention or treatment of coronavirus infection containing the antibody or antigen-binding fragment, are provided.

## Description

### [Technical Field]

The present invention relates to an antibody or antigen-binding fragment thereof against the causative virus (SARS-CoV-2) of novel coronavirus infection (COVID-19). The present invention also relates to therapeutic and prophylactic agents for coronaviruses using the antibody.

### [Background Art]

As of August 9, 2020, a total of 48,907 people had been infected with the new coronavirus infection (COVID-19) in Japan, of which 1,048 have died. The number of infected people is reported to be 14,953. Worldwide, 19.46 million people have been infected and 720,000 have died. COVID-19 has had a major impact on the global economy, and the inability to see an exit from the era of COVID-19 has become a major social problem. Thus, the development of vaccines and therapeutic methods against the novel coronavirus (SARS-CoV-2) is the most important global issue.

So far, various antiviral drugs have been studied, and remdesivir, which was developed for Ebola hemorrhagic fever, was urgently approved as a therapeutic agent for COVID-19, but there is no established effective therapeutic method. On the other hand, among the cases infected with SARS-CoV-2 and recovering after exacerbation once, there are cases with strong neutralizing antibodies in the plasma, and therapeutic methods using such convalescent plasma are being explored (convalescent plasma therapy) (Non-Patent Document 1: Shen C. et al., JAMA, 2020; 323 (16): 1582-1589, Non-Patent Document 2: Duan K. et al., Proc Natl Acad Sci, 2020; 117(17): 9490-9496). As shown there, many cases treated with plasma containing neutralizing antibodies show clinical recovery within days, including reduction in viral load, fever reduction, improvement in oxygen concentration, and the like, suggesting the usefulness of neutralizing antibodies. However, the plasma of convalescent cases containing high neutralizing antibodies is limited, and application to many cases is difficult.

On the other hand, there is a suggestion on a therapeutic method for suppressing exacerbation using the neutralizing antibody, by isolating the neutralizing antibody gene from B cells of COVID-19 convalescent cases and producing a large amount of the recombinant antibodies. Such efforts have been carried out in China, the United States, and Europe, and multiple papers have been published. Different papers use different neutralization experimental systems, making simple comparison difficult, but multiple antibodies with high neutralizing activity have been reported. For example, antibodies P2C-1F11, P2B-2F6 and P2C-1A3 (Non-Patent Document 3: Ju, B. et al. Nature, 584, 115-119 (2020)), an antibody CB6 (Non-Patent Document 4: Shi, R. et al. Nature, 584, 120-124 (2020)), and antibodies C121, Cl35 and C144 (Non-Patent Document 5: Robbiani, D. F. et al. Nature, 584, 437-442 (2020)) have been reported.

In addition, it has been reported that the spike protein of SARS-CoV-2 binds to angiotensin-converting enzyme 2 (ACE2) of target cells, and then fusion of the viral and cell membranes is induced by the action of the cell surface protease TMPRSS2 to establish infection (Non-Patent Document 6: Kai K. et al. Science, 2020; 367: 1412-1413). Furthermore, structural studies of the spike protein have revealed that its receptor binding domain (RBD) is the region that binds to ACE2, and it is considered that among the spike proteins, the RBD is the most important target of neutralizing antibodies (Non-Patent Document 7: Wrapp et al. Science, 2020; 367: 1260-1263).

In addition, an antibody that binds to the spike protein of human severe acute respiratory syndrome coronavirus (SARS-CoV) and neutralizes the virus has been reported (for example, Patent Document 1: JP-A-2008-529504, Patent Document 2: JP-A-2009-537143).

Since the end of 2020, the COVID-19 pandemic that occurred in 2020 has entered a new phase of the spread of infection with SARS-CoV-2 mutant strain. Mutant strains reported in the United Kingdom and the Republic of South Africa have also been confirmed in Japan (announced by the Ministry of Health, Labour and Welfare; March 03, 2021). These mutant strains have been reported to be resistant to antibodies induced by currently used vaccines and neutralizing antibodies urgently approved in the United States (Non-Patent Document 8: Wang P. et al., bioRxiv preprint doi: https://doi.org/10.1101/2021.01.25.428137). As a representative of mutant strains, the mutant strain B.1.1.7 accounting for the majority of new cases in the UK, the Danish mutant strain mink cluster 5, the South African mutant strain B.1.351, the Brazil mutant strain P.1, the Indian mutant strains B.1.167.1 and B.1.167.2, etc. are well known. The sequences of these mutant strains have been published and carry mutations in the spike protein.

### [Citation List]

### [Patent Document]

Patent Document 1: JP Laid-Open 2008-529504
Patent Document 2: JP Laid-Open 2009-537143

### [Non-Patent Document]

Non-Patent Document 1: Shen C.et al., JAMA, 2020; 323(16):1582-1589
Non-Patent Document 2: Duan K. et al., Proc Natl Acad Sci, 2020; 117 (17) : 9490-9496
Non-Patent Document 3: Ju, B. et al. Nature, 584, 115-119(2020)
Non-Patent Document 4: Shi, R. et al. Nature, 584, 120-124 (2020)
Non-Patent Document 5: Robbiani, D. F. et al. Nature, 584, 437-442(2020)
Non-Patent Document 6: Kai K. et al. Science, 2020;367:1412-1413
Non-Patent Document 7: Wrapp et al. Science, 2020;367:1260-1263
Non-Patent Document 8: Wang P.et al., bioRxiv preprint doi: https://doi.org/10.1101/2021.01.25.428137

### [Summary of the Invention]

### [Technical Problem]

It is an object of the present invention to provide an antibody or antigen-binding fragment thereof against the coronavirus (SARS-CoV-2). The present invention also provides a pharmaceutical composition against coronavirus infection using the antibody or antigen-binding fragment thereof.

### [Solution to Problem]

The present invention is based on the isolation of human monoclonal antibodies (mAbs) from IgG memory B cells harvested from COVID-19 infected and recovered patient donors. The present inventors have conducted intensive studies with the aim of developing a therapeutic method for suppressing exacerbation using a neutralizing antibody, by isolating antibody genes that produce antibodies neutralizing SARS-CoV-2 from B cells of COVID-19 convalescent cases and producing recombinant antibodies using them. As a result, four antibodies with strong neutralizing activity: CTMA9-105, CSSA10-121, CSSA8-92, and CSSA3-5 have been isolated from cases infected with coronavirus (SARS-CoV-2) and recovered after exacerbation, and their gene sequences have been identified, completing the present invention. As described herein, the inventors have used recombinant techniques to generate antibodies with improved properties. The antibody or antigen-binding fragment thereof of the present invention is characterized by binding to the RBD of the SARS-CoV-2 spike protein and neutralizing the SARS-CoV-2 virus. The antibody or antigen-binding fragment thereof of the present invention has superior SARS-CoV-2 virus-neutralizing activity and may be more useful than conventional antibodies. The present invention includes the following aspects.

[1] An antibody or antigen binding fragment thereof that binds to the Receptor Binding Domain (RBD) present in the spike protein of the coronavirus (SARS-CoV-2) and is capable of neutralizing the SARS-CoV-2 virus,
   comprising the following heavy chains CDR1-3 and light chains CDR1-3:
   (1) A heavy chain CDR1 selected from the group consisting of a heavy chain CDR1 of SEQ ID NO: 9 (GITVSSNY), a heavy chain CDR1 of SEQ ID NO: 12 (GLTVSRNY), a heavy chain CDR1 of SEQ ID NO: 14 (EITVSRNY), a heavy chain CDR1 of SEQ ID NO: 17 (GFTVSSNY), a heavy chain CDR1 consisting of a sequence in which one or two amino acids are deleted, substituted or added in any of heavy chains CDR1 selected from the group consisting of SEQ ID NOs: 9, 12, 14 and 17, and a heavy chain CDR1 having 90% or more substantial identity with any of heavy chains CDR1 selected from the group consisting of SEQ ID NOs: 9, 12, 14 and 17,
   (2) A heavy chain CDR2 selected from the group consisting of a heavy chain CDR2 of SEQ ID NO: 10 (IYSGGST), a heavy chain CDR2 of SEQ ID NO: 15 (IYSGGSR), a heavy chain CDR2 consisting of a sequence in which one or two amino acids are deleted, substituted or added in any of heavy chains CDR2 selected from the group consisting of SEQ ID NOs: 10 and 15, and a heavy chain CDR2 having 90% or more substantial identity with any of heavy chains CDR2 selected from the group consisting of SEQ ID NOs: 10 and 15,
   (3) A heavy chain CDR3 selected from the group consisting of a heavy chain CDR3 of SEQ ID NO: 11 (ARDLEEAGGMDV), a heavy chain CDR3 of SEQ ID NO: 13 (ARPIVGARAGMDV), a heavy chain CDR3 of SEQ ID NO: 16 (ARDKNEGAMDV), a heavy chain CDR3 of SEQ ID NO: 18 (ARSYGDYFIDY), a heavy chain CDR3 consisting of a sequence in which one, two or three amino acids are deleted, substituted or added in any of heavy chains CDR3 selected from the group consisting of SEQ ID NOs: 11, 13, 16 and 18, and a heavy chain CDR3 having 90% or more substantial identity with any of heavy chains CDR3 selected from the group consisting of SEQ ID NOs: 11, 13, 16 and 18,
   (4) A light chain CDR1 selected from the group consisting of a light chain CDR1 of SEQ ID NO: 19 (QSVPSIY), a light chain CDR1 of SEQ ID NO: 22 (QDISNY), a light chain CDR1 of SEQ ID NO: 25 (QGISSY), a light chain CDR1 of SEQ ID NO: 28 (QSVSSY), a light chain CDR1 consisting of a sequence in which one or two amino acids are deleted, substituted or added in any of light chains CDR1 selected from the group consisting of SEQ ID NOs: 19, 22, 25 and 28, and a light chain CDR1 having 90% or more substantial identity with any of light chains CDR1 selected from the group consisting of SEQ ID NOs: 19, 22, 25 and 28,
   (5) A light chain CDR2 selected from the group consisting of a light chain CDR2 of SEQ ID NO: 20 (GAS), a light chain CDR2 of SEQ ID NO: 23 (DAS), a light chain CDR2 of SEQ ID NO: 26 (AAS), and a light chain CDR2 consisting of a sequence in which one amino acid is deleted, substituted or added in any of light chains CDR2 selected from the group consisting of SEQ ID NOs: 20, 23 and 26, and
   (6) A light chain CDR3 selected from the group consisting of a light chain CDR3 of SEQ ID NO: 21 (QQYGSSPGT), a light chain CDR3 of SEQ ID NO: 24 (QQYDNLPVT), a light chain CDR3 of SEQ ID NO: 27 (QHLNSYSYT), a light chain CDR3 of SEQ ID NO: 29 (QQYGSSPWWT), a light chain CDR3 consisting of a sequence in which one or two amino acids are deleted, substituted or added in any of light chains CDR3 selected from the group consisting of SEQ ID NOs: 21, 24, 27 and 29, and a light chain CDR3 having 90% or more substantial identity with any of light chains CDR3 selected from the group consisting of SEQ ID NOs: 21, 24, 27 and 29.
[2] The antibody or antigen-binding fragment thereof described in [1], wherein the heavy chain CDR1 is a heavy chain CDR1 selected from the group consisting of SEQ ID NOs: 9, 12, 14 and 17, the heavy chain CDR2 is a heavy chain CDR2 selected from the group consisting of SEQ ID NOs: 10 and 15, the heavy chain CDR3 is a heavy chain CDR3 selected from the group consisting of SEQ ID NOs: 11, 13, 16 and 18, the light chain CDR1 is a light chain CDR1 selected from the group consisting of SEQ ID NOs: 19, 22, 25 and 28, the light chain CDR2 is a light chain CDR2 selected from the group consisting of SEQ ID NOs: 20, 23 and 26, and the light chain CDR3 is a light chain CDR3 selected from the group consisting of SEQ ID NOs: 21, 24, 27 and 29.
[3] The antibody or antigen-binding fragment thereof described in [1], wherein the heavy chain CDR1 is a heavy chain CDR1 of SEQ ID NO: 9, the heavy chain CDR2 is a heavy chain CDR2 of SEQ ID NO: 10, the heavy chain CDR3 is a heavy chain CDR3 of SEQ ID NO: 11, the light chain CDR1 is a light chain CDR1 of SEQ ID NO: 19, the light chain CDR2 is a light chain CDR2 of SEQ ID NO: 20, and the light chain CDR3 is a light chain CDR3 of SEQ ID NO: 21.
[4] The antibody or antigen-binding fragment thereof described in [1], wherein the heavy chain CDR1 is a heavy chain CDR1 of SEQ ID NO: 12, the heavy chain CDR2 is a heavy chain CDR2 of SEQ ID NO: 10, the heavy chain CDR3 is a heavy chain CDR3 of SEQ ID NO: 13, the light chain CDR1 is a light chain CDR1 of SEQ ID NO: 22, the light chain CDR2 is a light chain CDR2 of SEQ ID NO: 23, and the light chain CDR3 is a light chain CDR3 of SEQ ID NO: 24.
[5] The antibody or antigen-binding fragment thereof described in [1], wherein the heavy chain CDR1 is a heavy chain CDR1 of SEQ ID NO: 14, the heavy chain CDR2 is a heavy chain CDR2 of SEQ ID NO: 15, the heavy chain CDR3 is a heavy chain CDR3 of SEQ ID NO: 16, the light chain CDR1 is a light chain CDR1 of SEQ ID NO: 25, the light chain CDR2 is a light chain CDR2 of SEQ ID NO: 26, and the light chain CDR3 is a light chain CDR3 of SEQ ID NO: 27.
[6] The antibody or antigen-binding fragment thereof described in [1], wherein the heavy chain CDR1 is a heavy chain CDR1 of SEQ ID NO: 17, the heavy chain CDR2 is a heavy chain CDR2 of SEQ ID NO: 10, the heavy chain CDR3 is a heavy chain CDR3 of SEQ ID NO: 18, the light chain CDR1 is a light chain CDR1 of SEQ ID NO: 28, the light chain CDR2 is a light chain CDR2 of SEQ ID NO: 20, and the light chain CDR3 is a light chain CDR3 of SEQ ID NO: 29.
[7] The antibody or antigen-binding fragment thereof described in [1], which has a heavy chain variable region set forth in SEQ ID NO: 1 and a light chain variable region set forth in SEQ ID NO: 5.
[8] The antibody or antigen-binding fragment thereof described in [1], which has a heavy chain variable region set forth in SEQ ID NO: 2 and a light chain variable region set forth in SEQ ID NO: 6.
[9] The antibody or antigen-binding fragment thereof described in [1], which has the heavy chain variable region set forth in SEQ ID NO: 3 and the light chain variable region set forth in SEQ ID NO: 7.
[10] The antibody or antigen-binding fragment thereof described in [1], which has a heavy chain variable region as set forth in SEQ ID NO: 4 and a light chain variable region as set forth in SEQ ID NO: 8.
[11] The antibody or antigen-binding fragment thereof described in any one of [1] to [10], wherein in a neutralization assay using pseudoviruses, the antibody activity in neutralizing the SARS-CoV-2 virus has a high neutralizing ability expressed as 50% inhibitory concentration (IC₅₀ µg/mL) in a range from about 0.001 µg/mL to about 1 µg/mL, preferably from about 0.002 µg/mL to about 0.5 µg/mL, more preferably from about 0.002 µg/mL to about 0.1 µg/mL.
[12] The antibody or antigen-binding fragment thereof described in any one of [1] to [10], showing neutralizing activity against at least one SARS-CoV-2 mutant strain selected from the group consisting of B.1.1.7 mutant strain, mink cluster 5 mutant strain, B.1.351 mutant strain, P.1 mutant strain, P.3 mutant strains, B.1.617 mutant strain(including B.1.617.1 mutant strain and B.1.617.2 mutant strain), R.1 mutant strain, and B.1.427/B.1.429 mutant strains.
[13] The antibody or antigen-binding fragment thereof described in any one of [1] to [10], showing neutralizing activity against a SARS-CoV-2 mutant strain having one or more mutations selected from the group consisting of K417T/N mutation, E484K mutation, N501Y mutation, and L452R mutation.
[14] The antibody or antigen-binding fragment thereof described in any one of [1] to [10], selected from the group consisting of immunoglobulin molecules, monoclonal antibodies, human antibodies, chimeric antibodies, CDR-grafted antibodies, Fab, Fab', F(ab')2, Fd, Fv, disulfide-bound Fv, scFv, single domain antibodies, nanobody antibodies, diabody antibodies, bispecific antibodies, and multi-specific antibodies.
[15] A nucleic acid encoding the antibody or antigen-binding fragment thereof described in any one of [1]-[14].
[16] A vector comprising the nucleic acid described in [15] .
[17] A host cell comprising the nucleic acid described in [15] or the vector described in [16].
[18] A method for producing the antibody or antigen-binding fragment thereof described in any one of [1] to [14], comprising a step of culturing the host cell described in [15] under conditions suitable for expressing the antibody or antigen-binding fragment thereof.
[19] A pharmaceutical composition comprising the antibody or antigen-binding fragment thereof described in any one of [1] to [14] and a pharmaceutically acceptable carrier.
[20] A pharmaceutical composition for the prevention or treatment of coronavirus infection (preferably SARS-CoV-2 virus infection) in a subject, comprising the antibody or antigen-binding fragment thereof described in any one of [1] to [14].
[21] Use of the antibody or antigen-binding fragment thereof described in any one of [1] to [14] in the production of a pharmaceutical composition for the prevention or treatment of coronavirus infection (preferably SARS-CoV-2 virus infection) in a subject.
[22] The pharmaceutical composition described in [19] or [20], wherein it is administered in combination with another anti-coronaviral drug.
[23] The pharmaceutical composition described in [22], wherein the anti-coronaviral drug is at least one anti-coronaviral drug selected from remdesivir, favipiravir, dexamethasone, ciclenisonide, nafamostat, camostat, ivermectin, bamilanibimab, etesevimab, casilibimab, imdevimab, and HIV protease inhibitors (for example, Lopinavir, ritonavir or their combination, nelfinavir).

### [Advantageous Effect of the Invention]

The present invention provides neutralizing antibodies against the novel SARS-CoV-2 virus.

### [Brief Description of Drawings]

FIG. 1 shows the amino acid sequences of the heavy chain variable regions of four isolated antibodies (CTMA9-105HC, CSSA110-121HC, CSSA8-92HC, CSSA3-5HC), and the frame regions and CDR1 to 3 regions in each heavy chain variable region.
FIG. 2 shows the amino acid sequences of the light chain variable regions of four isolated antibodies (CTMA9-105HC, CSSA110-121HC, CSSA8-92HC, CSSA3-5HC), and the frame regions and CDR1 to 3 regions in each light chain variable region.
FIG. 3 schematically shows a system in which 293T cells are transfected with a plasmid co-expressing the SARS-CoV-2 spike protein and EGFP to express the spike protein on the cell surface.
FIG. 4 shows the analysis results by flow cytometry using the plasma of patients infected with COVID-19 and recovered. The upper figure shows the result using patient plasma, and the lower figure shows the result using IgG of a healthy person who is not infected. Arrows indicate spike protein binding activity.
FIG. 5 shows part of the results of evaluating the binding of recombinant IgG (rIgG) cloned in Example 2 to the spike protein receptor binding domain (RBD) of SARS-CoV-2 using the AlphaScreen system. The data show RBD binding activity in 7 antibodies, including 4 of the present invention. 10-121 indicates the culture supernatant and p10-121 indicates purified IgG (10 ng). PGN is a negative control. Specimens not labeled as "CTMA" are from CSSA.
FIG. 6 is a schematic of a pseudovirus neutralization test to measure SARS-CoV-2 neutralization activity.
FIG. 7 shows the results of measuring the binding activity of the four screened neutralizing antibodies to the spike protein of SARS-CoV-2.
FIG. 8 shows the results of measuring the neutralizing activity against a pseudovirus expressing the SARS-CoV-2 spike protein on the cell surface using the four neutralizing antibodies screened.
FIG. 9 shows an outline of cell fusion activity measurement. Cell fusion occurs when the spike protein of SARS-CoV-2 expressed on the surface of effector cells binds to ACE2 expressed on the surface of target cells. When cell fusion occurs, the DSP of each cell binds and exhibits activity.
FIG. 10 shows the results of measuring inhibitory activity of SARS-CoV-2 spike protein-mediated cell fusion using the effector cells shown in FIG. 9 and two types of target cells.
FIG. 11 shows amino acid mutations of B.1.1.7 mutant strain, mink cluster 5 mutant strain, B.1.351 mutant strain, and P.1 mutant strain, which are SARS-CoV-2 mutant strains with mutations in the spike protein.
FIG. 12 shows the results of measurement of neutralizing activity when cells are infected with pseudoviruses having the spikes of SARS-CoV-2 mutant strains (B.1.1.7 strain, B.1.351 strain, P.1 strain, mink cluster 5 strain) using neutralizing antibodies.
FIG. 13 shows the results of comparing the neutralizing activity against SARS-CoV-2 wild strain and SARS-CoV-2 mutant strain. Neutralizing activity is indicated by IC₅₀ [µg/mL]. Parentheses indicate the fold change compared to the neutralizing activity against wild strain.
FIG. 14 shows the results of measuring the neutralizing activity against the SARS-CoV-2 Indian mutant strain (B.1.617.2 strain) using neutralizing antibodies 9-105 and 10-121.
FIG. 15 shows the results of measuring the neutralizing activity against SARS-CoV-2 mutant strains (B.1.1.7 strain, B.1.351 strain, P.1 strain, B.1.617.1 strain, and B.1.617.2 strain) using the neutralizing antibody 9-105.
FIG. 16 shows the results of measuring the neutralizing activity against SARS-CoV-2 mutant strains (B.1.1.7 strain, B.1.351 strain, P.1 strain, B.1.617.1 strain, and B.1.617.2 strain) using the neutralizing antibody 10-121.

### [Description of Embodiments]

Hereinafter, the present invention will be illustrated in more detail along with preferred methods and materials which can be used in practice of the present invention. However the present invention is not limited to the following embodiments. Unless otherwise specified in the sentences, any technical terms and scientific terms used in the present specification have the same meaning as those generally understood by those of ordinary skill in the art to which the present invention belongs. Any materials and methods equivalent or similar to those described in the present specification can be used for practicing the present invention. All publications and patents cited herein in connection with the present invention described herein are incorporated by reference, for example, as indicating methodology, materials, etc. that can be used in the present invention.

In the present specification, the description of "A to B" indicating a numerical range means a numerical range including A and B as endpoints. The same applies to "A through B". Moreover, in the present specification, the term "about" is used in the sense of allowing ±10%. As used herein, "one or more" means "one, two, three, four or more" unless the meaning is explicitly indicated or the meaning is clear from the surrounding context.

In the present specification, three-letter or one-letter codes are used including the code "Xaa" or "X" to indicate any amino acid residue.

"SARS-CoV-2", also called "novel coronavirus", refers to a newly emerging coronavirus first isolated in Wuhan, China in 2020 and identified as the cause of an outbreak of severe acute respiratory illness. It binds to the human host cell receptor angiotensin-converting enzyme 2 (ACE2) via the viral spike protein. Several subspecies of SARS-CoV-2 spike protein have been reported, such as Wuhan type (Wuhan type S), European type (European type S_{D 6 1 4 G}), and the like. In the present specification, when the expression SARS-CoV-2 spike protein is used, this expression is used to include not only the Wuhan type (Wuhan type S) and the European type (European type S_{D614G}), but also any type of spike protein, unless explicitly stated or apparent from the surrounding context.

"SARS-CoV-2 spike protein", "spike protein" or "SARS-CoV-2(S)" means the spike protein of SARS-CoV-2 coronavirus. The SARS-CoV-2 spike protein is a 1273 amino acid type I membrane glycoprotein that forms a trimer and exists as a spike (peplomer) protruding from the surface of the SARS-CoV-2 virus particle. The SARS-CoV-2 spike protein has host receptor binding and membrane fusion functions and binds to ACE2 through an approximately 215 amino acid long receptor binding domain (RBD) present in the S1 subunit. The amino acid sequence of full-length SARS-CoV-2 spike protein (SEQ ID NO: 30) has been deposited in GeneBank under accession number YP_009724390. In the present specification, the SARS-CoV-2 spike protein includes recombinant SARS-CoV-2 spike proteins or fragments thereof.

"ACE2" is angiotensin converting enzyme 2, the receptor to which SARS-CoV-2 binds. ACE2 is an 805 amino acid type I transmembrane glycoprotein present on the cell surface that segregates angiotensin II into angiotensin 1-7 polypeptides. The isolated polypeptide has effects such as cardioprotection, vasodilation, and the like. ACE2 as used herein means human ACE2 unless otherwise specified. ACE2 acts as a functional receptor for the SARS-CoV-2 virus. Viruses bind ACE2 through their RBD. It has been reported that TMPRSS2, a serine transmembrane protease present on the host cell membrane, is required for binding of ACE2 to a virus and intracellular entry via membrane fusion.

Aspects of the present invention are described below, but the present invention is not limited thereto.

One aspect of the present invention is an antibody or antigen-binding fragment thereof that binds to the SARS-CoV-2 spike protein and is useful for binding to the SARS-CoV-2 spike protein to neutralize the virus. Hereinafter, in the present specification, the antibody and antigen-binding fragment thereof that bind to the SARS-CoV-2 spike protein are collectively referred to as "antibody and the like against SARS-CoV-2", "antibody and the like against the SARS-CoV-2 spike protein", "anti-SARS-CoV-2(S) antibody and the like", or simply as "antibody and the like" or "antibody" of the present invention, but if the text clearly refers to either an antibody or an antigen-binding fragment thereof, or if it can be clearly understood from the surrounding context that it is one of them, they are understood to mean either antibodies that bind the spike protein of SARS-CoV-2 or fragments that bind the spike protein of SARS-CoV-2.

As used herein, the term "antibody" is used in the sense of including complete antibodies and fragments thereof, but when the sentence clearly indicates either a complete antibody or an antibody fragment, or from the context before and after, where either of these can be clearly understood, it is understood to mean either an antibody or a fragment of an antibody. It is clear to those skilled in the art that antigen-binding fragments (fragments of antibodies that bind to antigens) can compete with intact antibodies for specific binding to antigens and exhibit similar effects of antibodies. See Fundamental Immunology, Chapter 7 (Paul, W., ed., 2nd ed., Raven Press, NY (1989)).

In certain embodiments, the antibody of the present invention is useful for inhibiting the binding of the virus to its host cell receptor, angiotensin-converting enzyme 2 (ACE2), and preventing entry of the SARS coronavirus into host cells. In certain embodiments, the antibody of the present invention exhibits antiviral activity by inhibiting SARS-CoV-2 spike protein-mediated cell fusion. In certain other embodiments, the antibody of the present invention prevents, treats, or ameliorates at least one symptom of SARS-CoV-2 virus infection in a subject (preferably a human). In certain other embodiments, the antibody of the present invention is prophylactically or therapeutically administered to a subject (preferably a human) infected with or at risk of being infected with the SARS-CoV-2 virus.

The antibody of the present invention can be full-length (e.g., IgG1, IgG2 or IgG4), or can include an antigen-binding portion thereof (e.g., Fab, Fab', F(ab')2, Fd, Fv, disulfide Fv, scFv, single domains, nanobodies, diabodies), which may be modified to improve functionality or to remove nonessential effector functions. Also, in certain embodiments, the antibody of the present invention can be bispecific or multispecific.

In one aspect, the present invention provides an isolated recombinant monoclonal antibody or antigen-binding fragment thereof that specifically binds to the spike protein of SARS-CoV-2. In certain embodiments, the antibody of the present invention is a fully human monoclonal antibody. The antibody and the like of the present invention bind to an epitope within the receptor binding domain (RBD) of the spike protein of SARS-CoV-2. In certain embodiments, the antibody and the like of the present invention bind to one or a plurality of amino acids selected from amino acids 322-536 (corresponding to the biotinylated RBD sequences provided by Professor Takao Hashiguchi, Kyushu University used in examples) of the SARS-CoV-2 spike protein (registered in GenBank as accession number YP_009724390) (SEQ ID NO: 30). In addition, the whole genome information of SARS-CoV-2 virus is registered in GeneBank as Genome Reference Sequence: NC_045512. In certain embodiments, the antibody and the like of the present invention bind to the Wuhan SARS-CoV-2 spike protein (Wuhan type S). In another embodiment, the antibody and the like of the present invention bind to the European SARS-CoV-2 spike protein (European type S_{D 6 1 4 G}). In yet another embodiment, the antibody and the like of the present invention bind to the SARS-CoV-2 spike proteins of various mutant strains. Representative mutant strains include, but are not limited to, the mutant strain B.1.1.7 lineage derived from the United Kingdom, the mutant strain B.1.351 lineage derived from South Africa, the mutant strain P.1 lineage found in travelers from Brazil in Japan, the mutant strain P.3 lineage derived from the Philippines, the mutant strain B.1.617 lineages derived from India (e.g., B.1.617.1 mutant strain and B.1.617.2 mutant strain), the mutant strain R.1 lineage with E484K mutation, and the B.1.427/B.1.429 lineages derived from the United States. The mutant strains also include the B.1.1.316 lineage with E484K mutation, the B.1.1.214 lineage which is the mainstreams in Japan, as well as lineages in which further mutations (e.g., E484K mutation) have occurred in them. The mutant strains can further include strains with 2-3 characteristic mutations in RBD, and characteristic mutations include, but not limited to, E484K, N501Y, K417T/N, and L452R mutations. In addition, mutant strains reported based on the region and country of origin include the Indian type (delta strain), the British type (alpha strain), the South African type (beta strain), the Brazilian type (gamma strain), and the Californian type (epsilon strain), the strain detected in Brazil (zeta strain), the strain detected in the United Kingdom (eta strain), the strain detected in Philippines (theta strain), the strain detected in New York (iota strain), the strain detected in India (kappa strain), the strain detected in South America such as Peru, Argentina and the like (lambda strain), etc., and the antibody and the like of the present invention can also be used against these mutant strains.

Representative anti-SARS-CoV-2 antibodies of the present invention include, for example, antibodies having amino acid sequences set forth in SEQ ID NOs: 1, 2, 3 or 4 in their heavy chain variable regions. Representative anti-SARS-CoV-2 antibodies of the present invention include, for example, antibodies having amino acid sequences set forth in SEQ ID NO: 5, 6, 7 or 8 in the light chain variable region. More representative anti-SARS-CoV-2 antibodies of the present invention include, for example, antibodies having combinations of SEQ ID NOs: 1 and 5, SEQ ID NOs: 2 and 6, SEQ ID NOs: 3 and 7, or SEQ ID NOs: 4 and 8 as a set of amino acid sequences of the heavy chain variable region and the light chain variable region.

The amino acid sequences of the heavy chain variable region (HCVR), the light chain variable region (LCVR), the heavy chain complementarity determining regions (HCDR1, HCDR2, and HCDR3), and the light chain complementarity determining regions (LCDR1, LCDR2 and LCDR3) of four representative anti-SARS-CoV-2 antibodies of the present invention are listed in the table below.

**Table 1**

| Antibody Name | HCVR | HCDR1 | HCDR2 | HCDR3 | LCVR | LCDR1 | LCDR2 | LCDR3 |
|---|---|---|---|---|---|---|---|---|
| CTMA9-105 HC | SEQ ID NO: 1 | SEQ ID NO: 9 | SEQ ID NO: 10 | SEQ ID NO: 11 | SEQ ID NO: 5 | SEQ ID NO: 19 | SEQ ID NO: 20 | SEQ ID NO: 21 |
| CSSA10-121 HC | SEQ ID NO: 2 | SEQ ID NO: 12 | SEQ ID NO: 10 | SEQ ID NO: 13 | SEQ ID NO: 6 | SEQ ID NO: 22 | SEQ ID NO: 23 | SEQ ID NO: 24 |
| CSSA8-92 HC | SEQ ID NO: 3 | SEQ ID NO: 14 | SEQ ID NO: 15 | SEQ ID NO: 16 | SEQ ID NO: 7 | SEQ ID NO: 25 | SEQ ID NO: 26 | SEQ ID NO: 27 |
| CSSA3-5 HC | SEQ ID NO: 4 | SEQ ID NO: 17 | SEQ ID NO: 10 | SEQ ID NO: 18 | SEQ ID NO: 8 | SEQ ID NO: 28 | SEQ ID NO: 20 | SEQ ID NO: 29 |

The present invention provides an antibody or antigen-binding fragment thereof containing HCVR containing any of HCVR amino acid sequences listed in Table 1, or an amino acid sequence selected from sequences having at least 70%, 75% or 80% substantial identity, preferably at least 90%, 92% or 95% substantial identity, and more preferably at least 98% or 99% substantial identity thereto.

In addition, the present invention provides an antibody or antigen-binding fragment thereof containing LCVR containing any of LCVR amino acid sequences listed in Table 1, or an amino acid sequence selected from sequences having at least 70%, 75% or 80% substantial identity, preferably at least 90%, 92% or 95% substantial identity, and more preferably at least 98% or 99% substantial identity thereto.

In addition, the present invention provides an antibody or antigen-binding fragment thereof containing an HCVR and LCVR amino acid sequence pair (HCVR/LCVR), containing any of HCVR amino acid sequences listed in Table 1 and any of LCVR amino acid sequences listed in Table 1 paired therewith.

In addition, the present invention provides an antibody or antigen-binding fragment thereof containing a heavy chain CDR1 (HCDR1) containing any of four HCDR1 amino acid sequences listed in Table 1, or an amino acid sequence selected from sequences having at least 90% substantial identity thereto.

In addition, the present invention provides an antibody or antigen-binding fragment thereof containing a heavy chain CDR2 (HCDR2) containing any of two HCDR2 amino acid sequences listed in Table 1, or an amino acid sequence selected from sequences having at least 90% substantial identity thereto.

In addition, the present invention provides an antibody or antigen-binding fragment thereof containing a heavy chain CDR3 (HCDR3) containing any of four HCDR3 amino acid sequences listed in Table 1, or an amino acid sequence selected from sequences having at least 90% substantial identity, and preferably having at least 95% substantial identity thereto.

In addition, the present invention provides an antibody or antigen-binding fragment thereof containing a light chain CDR1 (LCDR1) containing any of four LCDR1 amino acid sequences listed in Table 1, or an amino acid sequence selected from sequences having at least 90% substantial identity thereto.

In addition, the present invention provides an antibody or antigen-binding fragment thereof containing a light chain CDR2 (LCDR2) containing an amino acid sequence selected from sequences having any of three LCDR2 amino acid sequences listed in Table 1.

In addition, the present invention provides an antibody or antigen-binding fragment thereof containing a light chain CDR3 (LCDR3) containing any of four LCDR3 amino acid sequences listed in Table 1, or an amino acid sequence selected from sequences having at least 90% substantial identity, and preferably having at least 95% substantial identity thereto.

In addition, the present invention provides an antibody or antigen-binding fragment thereof containing an HCDR3 and LCDR3 amino acid sequence pair (HCDR3/LCDR3), containing any of four HCDR3 amino acid sequences listed in Table 1 and four LCDR3 amino acid sequences listed in Table 1 paired therewith. According to certain embodiments, the present invention provides an antibody or antigen-binding fragment thereof containing an HCDR3/LCDR3 amino acid sequence pair contained in any of four representative anti-SARS-CoV-2(S) antibodies listed in Table 1. In certain embodiments, the HCDR3/LCDR3 amino acid sequence pair is selected from the group consisting of SEQ ID NOs: 11/21 (CTMA9-105HC), 13/24 (CSSA10-121HC), 16/27 (CSSA8-92HC) and 18/29 (CSSA3-5HC).

In addition, the present invention provides an antibody or antigen-binding fragment thereof containing a set of six CDRs (i.e., a set of HCDR1-HCDR2-HCDR3+LCDR1-LCDR2-LCDR3) contained in any of representative anti-SARS-CoV-2(S) antibodies listed in Table 1. In certain embodiments, the HCDR1-HCDR2-HCDR3+LCDR1-LCDR2-LCDR3 amino acid sequence set is selected from the group consisting of SEQ ID NOs: 9-10-11+19-20-21 (CTMA9-105HC), 12-10-13+22-23-24 (CSSA10-121HC), 14-15-16+25-26-27 (CSSA8-92HC) and 17-10-18+28-20-29 (CSSA3-5HC).

In another embodiment, the present invention provides an antibody or antigen-binding fragment thereof containing a combination of any of a set of three CDRs contained in the HCVR amino acid sequence (i.e., HCDR1-HCDR2-HCDR3) and any of a set of three CDRs contained in the LCVR amino acid sequence (i.e., the set of LCDR1-LCDR2-LCDR3) defined by any of representative anti-SARS-CoV-2 antibodies listed in Table 1. In certain embodiments, the set of HCDR1-HCDR2-HCDR3 is selected from the group consisting of sets of SEQ ID NOs: 9-10-11, 12-10-13, 14-15-16 and 17-10-18; and the set of LCDR1-LCDR2-LCDR3 is selected from the group consisting of sets of SEQ ID NOs: 19-20-21, 22-23-24, 25-26-27 and 28-20-29.

Methods of identifying CDRs within HCVR and LCVR amino acid sequences are well known in the art and can be used to identify CDRs within an identified HCVR and/or LCVR amino acid sequence. Typical examples used to identify CDR boundaries include, for example, the IMGT definition, the Kabat definition, the Chothia definition, the AbM definition, the Martin definition, the Gelfand definition, and the Honneger definition (Aho's definition). Public databases are also available for identifying CDR sequences within antibodies.

The present invention includes the anti-SARS-CoV-2(S) antibody and the like with modified glycosylation patterns. In certain embodiments, modifications can be made to remove unwanted glycosylation sites, e.g., antibodies lacking fucose moieties have enhanced antibody-dependent cellular cytotoxicity (ADCC). Also, in certain embodiments, galactosylation modifications can improve complement dependent cytotoxicity (CDC). Antibodies and the like having modifications on their sugar chains are also included in the present invention.

In addition, the present invention provides antibody and the like that compete with antibody and the like, containing HCVR CDRs and LCVR CDRs having amino acid sequences selected from the sequences listed in Table 1 for specific binding to the spike protein of SARS-CoV-2.

In addition, the present invention provides antibodies that block or reduce (preferably completely block) SARS-CoV-2 spike protein binding to ACE2. The antibodies that block or reduce (preferably completely block) binding of the SARS-CoV-2 spike protein to ACE2 may bind at or near the binding site of the SARS-CoV-2 spike protein to ACE2. In certain embodiments, the present invention provides antibodies that block or reduce (preferably completely block) binding of the SARS-CoV-2 spike protein to mammalian, preferably human ACE2.

In another aspect of the present invention, the present invention provides nucleic acid molecules encoding the anti-SARS-CoV-2(S) antibody and the like. For example, the present invention provides nucleic acid molecules encoding any of HCVR amino acid sequences listed in Table 1, and in certain embodiments, the nucleic acid molecules contain any of nucleic acid sequences encoding HCVR amino acid sequences described in Table 1, or a polynucleotide sequence selected from sequences having at least 90%, 92% or 95% sequence identity, and at least 98% or 99% sequence identity to it. The invention also provides, for example, nucleic acid molecules encoding any of LCVR amino acid sequences listed in Table 1, and in certain embodiments, the nucleic acid molecules contain any of nucleic acid sequences encoding LCVR amino acid sequences described in Table 1, or a polynucleotide sequence selected from sequences having at least 90%, 92% or 95% sequence identity, and at least 98% or 99% sequence identity to it.

In addition, the present invention provides nucleic acid molecules encoding any of HCDR1 amino acid sequences listed in Table 1, and the nucleic acid molecules contain any of nucleic acid sequences encoding HCDR1 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of HCDR2 amino acid sequences listed in Table 1, and the nucleic acid molecules contain any of nucleic acid sequences encoding HCDR2 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of HCDR3 amino acid sequences listed in Table 1, and the nucleic acid molecules contain any of nucleic acid sequences encoding HCDR3 amino acid sequences described in Table 1, or a polynucleotide sequence selected from sequences having at least 90%, 92% or 95% sequence identity, and at least 98% or 99% sequence identity to it.

In addition, the present invention provides nucleic acid molecules encoding any of LCDR1 amino acid sequences listed in Table 1, and the nucleic acid molecules contain any of nucleic acid sequences encoding LCDR1 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of LCDR2 amino acid sequences listed in Table 1, and the nucleic acid molecules contain any of nucleic acid sequences encoding LCDR2 amino acid sequences described in Table 1, or a polynucleotide sequence selected from sequences having at least 90%, 92% or 95% sequence identity, and at least 98% or 99% sequence identity to it. In addition, the present invention provides nucleic acid molecules encoding any of LCDR3 amino acid sequences listed in Table 1, and the nucleic acid molecules contain any of nucleic acid sequences encoding LCDR3 amino acid sequences described in Table 1, or a polynucleotide sequence selected from sequences having at least 90%, 92% or 95% sequence identity, and at least 98% or 99% sequence identity to it.

In addition, the present invention provides a nucleic acid molecule encoding an HCVR amino acid sequence, wherein the HCVR amino acid sequence contains a set of three CDR amino acid sequences (i.e., HCDR1-HCDR2-HCDR3), wherein the HCDR1-HCDR2-HCDR3 amino acid sequence set is defined by any of four representative anti-SARS-CoV-2(S) antibodies listed in Table 1. The invention also provides a nucleic acid molecule encoding an LCVR amino acid sequence, wherein the LCVR amino acid sequence contains a set of three CDR amino acid sequences (i.e., LCDR1-LCDR2-LCDR3), wherein the LCDR1-LCDR2-LCDR3 amino acid sequence set is defined by any of representative anti-SARS-CoV-2(S) antibodies listed in Table 1.

In addition, the present invention provides nucleic acid molecules encoding both HCVR and LCVR, wherein the nucleic acid molecules contain nucleic acid sequences encoding any of HCVR amino acid sequences listed in Table 1 and nucleic acid sequences encoding any of LCVR amino acid sequences listed in Table 1. The nucleic acid molecules contain any of nucleic acid sequences encoding HCVR amino acid sequences listed in Table 1, or a polynucleotide sequence selected from sequences having at least 90%, 92% or 95% sequence identity and at least 98% or 99% sequence identity to it, and any of nucleic acid sequences encoding LCVR amino acid sequences listed in Table 1, or a polynucleotide sequence selected from sequences having at least 90%, 92% or 95% sequence identity and at least 98% or 99% sequence identity to it.

The antibody and the like of the present invention contain the anti-SARS-CoV-2(S) antibody and the like that bind to the same epitope as any of antibodies having amino acid sequences of HCVR and LCVR listed in Table 1 that specifically bind to the SARS-CoV-2 spike protein obtained from four antibodies described in examples, or bind to at least a portion of that epitope. Such antibodies include antibodies and antigen-binding fragments thereof against the SARS-CoV-2 spike protein that cross-compete with any of antibodies having HCVR and LCVR amino acid sequences listed in Table 1.

By using methods known in the art, it can be easily determined whether antibodies (including complete antibodies and fragments thereof) bind to the same epitope as the reference anti-SARS-CoV-2(S) antibody and the like, or compete with the reference anti-SARS-CoV-2(S) antibody and the like for binding. For example, to determine if the test antibody binds to the same epitope as the reference anti-SARS-CoV-2(S) antibody and the like of the present invention, the reference antibody and the like are placed in conditions that allow them to bind to the SARS-CoV-2 spike protein under saturated conditions. The ability of the test antibody to bind to the SARS-CoV-2 spike protein is then assessed. If the test antibody is able to bind to the SARS-CoV-2 spike protein after saturation binding with the reference anti-SARS-CoV-2(S) antibody and the like, it is concluded that the test antibody binds to a different epitope than the reference anti-SARS-CoV-2(S) antibody and the like. On the other hand, if the test antibody is unable to bind to the SARS-CoV-2 spike protein after saturation binding with the reference anti-SARS-CoV-2(S) antibody and the like, it is concluded that the test antibody binds to the same epitope as that bound by the reference anti-SARS-CoV-2(S) antibody and the like. To determine if the test antibody competes with the reference anti-SARS-CoV-2(S) antibody for binding, it can be confirmed by testing for competition with each other. In a first test, the reference antibody and the like are allowed to bind to the SARS-CoV-2 spike protein under saturated conditions, and then the binding of the test antibody to the SARS-CoV-2 spike protein is assessed. In a second test, the test antibody is allowed to bind to the SARS-CoV-2 spike protein under saturated conditions, and then the binding of the reference antibody and the like to the SARS-CoV-2 spike protein is assessed. In both tests, if only the (saturated) antibody in the first test is capable of binding to the SARS-CoV-2 spike protein, then it is concluded that the test antibody and the reference antibody and the like compete for binding to the SARS-CoV-2 spike protein. As will be appreciated by those of skill in the art, an antibody that competes with the reference antibody and the like for binding will not necessarily bind to an epitope that matches the reference antibody, but will sterically inhibit the binding of the reference antibody and the like by binding to overlapping or adjacent epitopes. Thus, it is concluded that two antibodies bind to the same or overlapping epitopes if each competitively inhibits (blocks) the binding of the other to the antigen. Antibodies that compete with the antibody and the like of the present invention are also included in the present invention.

In one aspect, the present invention provides recombinant expression vectors capable of expressing the anti-SARS-CoV-2(S) antibody and the like. In another aspect, the present invention provides recombinant expression vectors capable of expressing polypeptides containing heavy and/or light chain variable regions of the anti-SARS-CoV-2(S) antibody. For example, the present invention contains recombinant expression vectors containing any of nucleic acid molecules described above, i.e., nucleic acid molecules encoding any of HCVR, LCVR, and/or CDR amino acid sequences listed in Table 1. Any expression vectors used in this technical field can be used without limitation, and such vectors are known to those skilled in the art. The antibody and the like of the present invention can be produced by culturing host cells into which such vectors have been introduced under conditions that allow the production of antibodies or antibody fragments, and recovering the produced antibodies and antibody fragments. Such methods are also included within the scope of the present invention.

In yet another aspect, the present invention provides a pharmaceutical composition containing a therapeutically effective amount of at least one antibody or antigen-binding fragment thereof that specifically binds to the SARS-CoV-2 spike protein, and a pharmaceutically acceptable carrier. In one embodiment, the present invention also includes a combined use of the anti-SARS-CoV-2(S) antibody and the like with other therapeutic agents, and a composition which is a compounding agent of the anti-SARS-CoV-2(S) antibody and the like and other therapeutic agents. A second therapeutic agent that is used in combination or blended with the antibody and the like of the present invention is any agent that is advantageously used in combination or blended with the anti-SARS-CoV-2(S) antibody. Typical agents that are advantageously used in combination or blended include, but not limited to, other agents that inhibit the activity of SARS-CoV-2 that bind to SARS-CoV-2 but do not cross-compete with the antibody and the like of the present invention (including other antibodies or antigen-binding fragments thereof, etc.), and/or agents that do not directly bind to SARS-CoV-2 but nevertheless inhibit viral activity, including infectivity of host cells. In certain embodiments, the second therapeutic agent is an agent that helps combat or reduce potential side effects, if any, associated with the antibody and the like of the present invention. The second therapeutic agents can be selected from the group consisting of, for example, anti-inflammatory agents (e.g., corticosteroids and non-steroidal anti-inflammatory agents), anti-infective agents, anti-viral agents, nutritional supplements such as antioxidants, and any other drugs and therapies known in the art. Examples thereof include preferably remdesivir, favipiravir, dexamethasone, ciclenisonide, nafamostat, camostat, ivermectin, bamilanibimab, etecevimab, casilibimab, imdevimab, and HIV protease inhibitors (e.g., lopinavir, ritonavir or combination drugs thereof, nelfinavir).

In another aspect, the present invention provides a method of treating or preventing a disease associated with SARS-CoV-2 in a subject (preferably human) using the anti-SARS-CoV-2(S) antibody or antigen-binding portion of the antibody of the present invention. Here, the treating or preventing method includes administering a pharmaceutical composition containing a therapeutically or prophylactically effective amount of the antibody and the like of the present invention to a subject (preferably a human) in need thereof. In one embodiment, the present invention provides methods of preventing, treating or ameliorating at least one symptom of SARS-CoV-2 infection, including administering a therapeutically or prophylactically effective amount of the anti-SARS-CoV-2(S) antibody and the like of the present invention to a subject (preferably a human) in need thereof. In certain embodiments, the present invention provides a method of ameliorating or reducing the severity of at least one symptom or sign of SARS infection in a subject by administering the anti-SARS-CoV-2(S) antibody and the like of the present invention. At least one symptom or sign is selected from the group consisting of inflammation in lungs, alveolar damage, fever, respiratory disorders (nasal congestion, runny nose, sore throat, cough, shortness of breath), headache, malaise, dysgeusia, olfactory disturbance, diarrhea, vomiting, hypercoagulability, thrombosis, Kawasaki disease-like vasculitis, renal dysfunction (e.g., nephritis), organ failure, pneumonia, septic shock and death. In certain other embodiments, the antibody and the like of the present invention are administered therapeutically or prophylactically to a subject infected with or at risk of being infected with SARS-CoV-2. Subjects (patients) at risk include, but not limited to, those with immunocompromised conditions, the elderly (aged 65 and over), those with underlying medical conditions such as pulmonary infections, heart disease, respiratory disease, diabetes or dialysis patients, medical workers, persons in close contact with persons with confirmed or suspected SARS infection, or persons expected to have contact with such persons. The antibody and the like of the present invention are administered intradermally, transdermally, intramuscularly, intraperitoneally, intravenously, subcutaneously, intranasally, epidurally, or orally. In one preferred embodiment, the antibody and the like of the present invention are administered as a single intravenous injection, taking into account the maximum concentration of the antibody in the subject's (patient's) plasma. In one embodiment, the antibody and the like of the present invention are administered at a dose of about 0.1 mg to about 100 mg/kg body weight of the subject. In certain other embodiments, the antibody and the like of the present invention are administered once or multiple times at a dose containing about 0.1 mg to about 100 mg/kg body weight of the subject.

The present invention also includes the use of the anti-SARS-CoV-2(S) antibody or antigen-binding fragment thereof of the present invention in the production of a medicament.

An antibody can be used in the sense of including immunoglobulin molecules containing four polypeptide chains, and two heavy (H) chains and two light (L) chains inter-connected by disulfide bindings (i.e., "complete antibody molecules"), multimers thereof (e.g., IgA or IgM), or antigen-binding fragments thereof. Each heavy chain of the antibody contains a heavy chain variable region (HCVR or VH) and a heavy chain constant region (including domains CH1, CH2 and CH3). Each light chain of the antibody contains a light chain variable region (LCVR or VL) and a light chain constant region (CL). HCVR and LCVR are further subdivided into framework regions (FR) and complementarity determining regions (CDR). Each HCVR and LCVR contains three CDRs and four FRs, arranged from amino-terminus to carboxy-terminus in the following order: FR1, CDR1, FR2, CDR2, FR3, CDR3, FR4. In embodiments of the antibody and the like of the present invention, the FRs of the antibody (or antigen-binding fragment thereof) may be identical to human germline sequences, or may be naturally or artificially modified.

An antibody can also have one or more CDR residues substituted or one or more CDRs omitted without losing its binding properties. Antibodies have also been reported that bind even with the omission of one or two CDRs. CDR residues that do not contact with an antigen can be identified by molecular modeling or experimentally. When substitutions of CDR residues are made, they may preferably be made at residues that do not contact with an antigen. The amino acid residues to be substituted and the positions for substitution within the CDRs are selected empirically based on a variety of factors. Substitutions can be conservative or non-conservative amino acid substitutions. A "conservative amino acid substitution" is one in which an amino acid residue is replaced by another amino acid residue having a side chain (R group) with similar chemical properties (e.g., charge or hydrophobicity), and in general, conservative amino acid substitutions do not substantially alter the functional properties of a protein (e.g., antigen-binding activity or binding properties). Examples of groups of amino acids with side chains with similar chemical properties include: 1) aliphatic side chains: glycine, alanine, valine, leucine and isoleucine; 2) aliphatic-hydroxyl side chains: serine and threonine; 3) amide-containing side chains: asparagine and glutamine; 4) aromatic side chains: phenylalanine, tyrosine and tryptophan; 5) basic side chains: lysine, arginine and histidine; 6) acidic side chains: aspartate and glutamate; and 7) sulfur-containing side chains: cysteine and methionine. Conservative amino acid substitutions refer to substitutions between amino acids within the same class, and non-conservative amino acid substitutions refer to exchanging members of one of these classes for members of another class. Preferred conservative amino acid substitutions are between: valine-leucine-isoleucine, phenylalanine-tyrosine, lysine-arginine, alanine-valine, glutamate-aspartate, and asparagine-glutamine.

The human anti-SARS-CoV-2(S) antibody and the like disclosed in the present specification include those having one or more amino acids substituted, added and/or deleted in the framework and/or CDR regions of the heavy and light chain variable domains compared to the corresponding sequences. The present invention includes antibodies and antigen-binding fragments thereof derived from any of amino acid sequences disclosed in the present specification, wherein within one or more framework and/or CDR regions, one or more amino acids may be conservatively substituted or mutated to the corresponding residues in the germline sequence from which the antibody is derived, or to the corresponding residues in another human germline sequence (such sequence alterations are referred to herein as "conservative mutation"). One skilled in the art can generate numerous antibodies and antigen-binding fragments containing one or more individual mutations or combinations thereof starting from the heavy and light chain variable region sequences disclosed in the present specification. The resulting antibodies and antigen-binding fragments can be readily identified and selected for one or more desired properties, such as improved binding specificity, increased binding affinity, reduced immunogenicity, and the like. Antibodies and antigen-binding fragments obtained by such general methods are also included in the present invention.

In one aspect, the amino acid substitutions for the anti-SARS-CoV-2(S) antibody and the like of the present invention give any one or more of (1) reducing susceptibility to proteolysis, (2) reducing susceptibility to oxidation, (3) changing binding affinity to form protein complexes, and (4) conferring or altering other physicochemical or functional properties, and are amino acid substitutions conserving specific binding to the SARS-CoV-2 spike protein. For example, one or more amino acid substitutions, preferably conservative amino acid substitutions, may be made in parts of the polypeptide, preferably outside the domains that form intermolecular contacts between the antigen and the antibody. Conservative amino acid substitutions should not substantially alter the structural features of the parent sequence; and for example, the substituted amino acids should not alter the antiparallel β-sheets that make up the immunoglobulin binding domain occurring in the parent sequence, or should not disrupt other secondary structures that characterize the parent sequence. Examples of secondary and tertiary structures of polypeptides known to those skilled in the art are described in, for example, Proteins, Structures and Molecular Principles (Creighton, Ed., W.H. Freeman and Company, New York (1984)); Introduction to Protein Structure (C. Branden and J. Tooze, eds., Garland Publishing, New York, N. Y. (1991)); and Thornton et al., Nature 354: 105 (1991), which are incorporated herein as a part of the present specification.

In addition, the present invention also includes the human anti-SARS-CoV-2(S) antibody and the like including variants of any of HCVR, LCVR, and/or CDR amino acid sequences disclosed in the present specification, having one or more conservative substitutions. For example, the present invention includes the anti-SARS-CoV-2(S) antibody and the like having no more than 10, no more than 8, no more than 6, no more than 4, or no more than 2 conservative amino acid substitutions related to any of HCVR, LCVR, and/or CDR amino acid sequences disclosed in the present specification.

The antibody in the present invention is preferably a human antibody. The human antibody refers to an antibody that has variable and constant regions derived from human germline immunoglobulin sequences. The antibodies exemplified in the present specification are human antibodies generated by in vitro activating B cells taken from patient donors who have recovered from SARS-CoV-2 infection. As used herein, "human antibody" is not intended to include monoclonal antibodies in which CDR sequences derived from the germline of another mammalian species (e.g., mouse) have been grafted onto human FR sequences, although it is meant to include antibodies produced recombinantly in non-human mammals or non-human mammalian cells. The antibodies described in examples of the present specification are human antibodies.

As used herein, "recombinant" means the antibody and the like of the present invention produced, expressed, isolated, or obtained by techniques or methods known in the art as recombinant DNA technology, including, for example, transgenic expression; and includes an antibody or antigen-binding fragment thereof expressed in a non-human mammal (including a transgenic non-human mammal, e.g., transgenic mouse), or mammalian cell (e.g., CHO cell) expression system, or isolated from recombinant combinatorial human antibody libraries.

As used herein, expressions such as "specifically binding" or "specifically bind to" mean that an antibody or an antigen-binding fragment thereof binds to an antigen under physiological conditions to form a complex. Specific binding is characterized by an equilibrium dissociation constant of at least about 1×10⁻⁸ M or less. Methods for determining whether two molecules specifically bind are well known in the art and include, for example, equilibrium dialysis, surface plasmon resonance, and the like, and it can be confirmed using these methods as appropriate. The surface plasmon resonance is identified, for example, by BIACORE (trademark).

As used herein, the terms "antigen-binding fragment" of an antibody, "antigen-binding fragment thereof" of an antibody, "fragment" of an antibody, and the like, include polypeptides or glycoproteins that specifically bind to the antigen SARS-CoV-2 spike protein to form a complex, that are optionally naturally occurring, obtained using enzymatic or biochemical means, synthesized, or obtained by genetic engineering. As used herein, the term refers to one or more fragments of an antibody that retains the ability to bind to the SARS-CoV-2 spike protein, and for example, may be produced by recombinant DNA techniques or enzymatically or chemically cleaving a complete antibody. As used herein, the term includes, but not limited to, for example, Fab, Fab', F(ab')2, Fd, Fv, disulfide Fv, dAb fragments, fragments containing complementarity determining regions (CDRs), single domains such as isolated CDR fragments, restricted FR3-CDR3-FR4 peptides, single chain antibodies (scFv), minibodies, nanobodies (e.g., monovalent nanobodies, bivalent nanobodies, etc.), diabodies (diabody), domain deficient antibodies, chimeric antibodies, CDR-grafted antibodies, bispecific antibodies, multispecific antibodies (e.g., trispecific antibody, tetraspecific antibody), polypeptides containing at least a portion of an antibody having specific antigen-binding ability, and other engineered molecules such as small modular immunodrugs.

Antigen-binding fragments of antibodies can be obtained using any suitable standard technique, such as, for example, protein digestion, or recombinant genetic engineering techniques involving the manipulation and expression of the DNA encoding the variable and (optionally) constant domains of the antibody, from a complete antibody molecule or its amino acid or genetic information. DNA is sequenced and chemical or molecular biological techniques are used, for example, to place one or more variable and/or constant domains in proper arrangement, alternatively, for example, arbitrary codons are introduced to create cysteine residues or to modify/add/delete amino acids, and other operation are performed, thereby any antigen-binding fragment can be produced.

An antigen-binding fragment of an antibody typically contains at least one variable domain. A variable domain can be of any size or amino acid composition and generally contains at least one CDR, which is flanked by or in-frame with one or more framework sequences. In antigen-binding fragments having a VH domain joined to a VL domain, the VH and VL domains are relatively positioned in any suitable arrangement. Antigen-binding fragments of antibodies include, for example, variable regions that are dimeric, VH-VH, VH-VL or VL-VL dimers. Alternatively, antigen-binding fragments of antibodies contain monomeric VH or VL domains.

In certain embodiments, an antigen-binding fragment of an antibody contains at least one variable domain covalently linked to at least one constant domain. Typical arrangements of variable and constant domains within the antigen-binding fragments of antibodies of the present invention include, but are not limited to: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any arrangement of variable and constant domains, including any of exemplary arrangements described above, the variable and constant domains are either directly linked to each other or linked by a linker region. The linker region consists of at least 2 (e.g., 5, 10, 15, 20, 40, 60 or more) amino acids and connect adjacent variable and/or constant domains. Additionally, the antigen-binding fragments of antibodies of the present invention contain homo-dimers or hetero-dimers (or other multimers) of any of the above variable and constant domain arrangements in which one or more monomeric VH or VL domains are non-covalently associated.

As with complete antibody molecules, antigen-binding fragments can be monospecific or multispecific (e.g., bispecific). A multispecific antigen-binding fragment of an antibody typically contains at least two different variable domains, each variable domain has an ability of specifically binding to different antigens or different epitopes on the same antigen. Any multispecific antibodies, including the bispecific antibodies in the present specification, can be produced using routine techniques available in the art and used as antigen-binding fragments of antibodies for the purposes of the present invention.

The antibody of the present invention is preferably an isolated antibody. As used herein, the "isolated antibody" means an antibody that is substantially free from other antibodies with different antigenic specificities.

As used herein, the terms "neutralizing antibody", "antibody that neutralizes SARS-CoV-2 viral activity", "antibody having an ability of neutralizing SARS-CoV-2 viral activity", etc. refer to an antibody wherein binding of the antibody to the SARS-CoV-2 spike protein results in inhibition of at least one biological activity of SARS-CoV-2, wherein the antibody and the like of the present invention prevent (reduce) or block binding of SARS-CoV-2 to ACE2, or prevent (reduce) or block infection of cells via binding of SARS-CoV-2 to ACE2 (i.e., inhibit cell membrane fusion). Preferably, the antibody and the like of the present invention completely block the binding of SARS-CoV-2 to ACE2.

The antibody and the like of the present invention have an ability of immunospecifically binding to the SARS-CoV-2 spike protein and neutralizing SARS-CoV-2 viral infection. The activity of the antibody and the like of the present invention is determined by in vitro or in vivo assays. The activity can be determined and measured based on the binding activity to the SARS-CoV-2 spike protein, preferably RBD of the spike protein. The activity can also be confirmed and measured by neutralizing activity against the SARS-CoV-2 virus. Preferably, the activity is measured by neutralizing activity. The ability of the antibody and the like of the present invention to bind SARS-CoV-2 and neutralize the activity of SARS-CoV-2 is measured using any standard methods known to those of skill in the art, including binding assays or neutralization assays, as described herein. Representative in vitro assays for measuring binding and blocking activity are described in examples of the present specification. The term "50% inhibitory concentration" (abbreviated as "IC₅₀") represents the concentration of the antibody of the present invention required for 50% neutralization of the SARS-CoV-2 virus. Lower IC₅₀ values have stronger neutralizing activity.

The term "TCID₅₀" refers to the amount of virus required to infect 50% of the cells in the tissue culture. 100x and 200x refer to 100 or 200 times the concentration of virus compared to TCID₅₀.

In certain embodiments, the antibody and the like of the present invention have a neutralizing ability expressed as 50% inhibitory concentration (IC₅₀ µg/ml) at the antibody concentrations within a range of about 0.001 µg/mL to about 1 µg/mL, or about 0.001 µg/mL to about 0.5 µg/mL, or about 0.002 µg/mL to about 0.25 µg/mL, or about 0.002 ug/mL to about 0.1 µg/mL in neutralizing the SARS-CoV-2 virus in the neutralization assay using a pseudovirus. The type of target cells used in the neutralization assay using a pseudovirus is not particularly limited, and any cells can be used as long as virus infection can be detected. Examples thereof include, but are not limited to, artificially produced cells (293F/ACE2/TMPRSS2 cells) shown in examples in the present specification, human lung cancer cells (alveolar epithelial cells, Calu-3), and gastrointestinal tract mucosal epithelial cells (Caco-2). Since the neutralizing activity may differ depending on the target cell, for example, the 50% inhibitory concentration can be defined as the neutralizing ability when using 293F/ACE2/TMPRSS2 cells. Antibodies used in the neutralization assays described in examples in the present specification show the 50% inhibitory concentration at about 0.25 µg/ mL for less active antibodies, and the 50% inhibitory concentration at about 0.002 µg/mL for more active antibodies. This indicates that the antibody of the present invention has a very high SARS-CoV-2 virus neutralizing activity.

In certain embodiments, the antibody and the like of the present invention preferably also have a neutralizing ability against at least one mutant strain of the SARS-CoV-2 virus. Mutant strains of the SARS-CoV-2 virus have mutations in any part of the sequence of SARS-CoV-2 virus, and especially those with mutations in spike protein sequence can be exemplified. Mutant stains include, but are not limited to, for example, the B.1.1.7 mutant strain, the mink cluster 5 mutant strain, the B.1.351 mutant strain, the P.1 strain mutant strain, the P.3 mutant strain, the B.1.617 mutant strains (e.g., B.1.617.1 mutant strain and B.1.617.2 mutant strain), the R.1 mutant strain, and the B.1.427/B.1.429 mutant strains. The mutant strain also includes mutant strains having at least one mutation selected from the group consisting of 417N/T, E484K, N501Y, and L452R in the spike protein, and for example, mutant strains having mutations of K417N and/or E484K are mentioned. The antibody and the like of the present invention preferably have a neutralizing ability expressed as 50% inhibitory concentration (IC₅₀ µg/ml) within a range of about 0.001 µg/mL to about 1 µg/mL, or about 0.001 µg/mL to about 0.5 µg/mL, or about 0.002 µg/mL to about 0.25 µg/mL, or about 0.002 µg/mL to about 0.1 µg/mL against any one of mutant strains of the SARS-CoV-2 virus in the neutralization assay using a pseudovirus.

As used herein, the term "epitope" refers to a site or region on an antigen that is an antigenic determinant that interacts with a specific antigen-binding site in the variable region of an antibody molecule. The epitopes is defined as either structural or functional. A functional epitope is generally a subset of a structural epitope and includes those residues that are directly involved in the affinity of the interaction. The epitope can also be conformational, including non-linear amino acid relationships. In certain embodiments, the epitope includes determinants that are chemically active surface groups of molecules such as amino acids, sugar side chains, phosphate groups or sulfonyl groups, and for example, has specific three dimensional structural features and/or specific residue modifications. When amino acid substitutions are made in the antibody and the like of the present invention, substitutions that do not affect the epitope-binding activity of the antibody or antigen-binding fragment thereof or that have little effect on the binding activity are preferred.

As used herein, the term "cross-compete" means that an antibody or antigen-binding fragment thereof binds to an antigen and inhibits or blocks the binding of another antibody or antigen-binding fragment thereof. In certain embodiments, the first and second antibodies bind to the same epitope. Alternatively, the first and second antibodies bind to different but overlapping epitopes such that binding of one antibody inhibits or blocks binding of the other antibody, e.g., through steric hindrance. Cross-competition between antibodies can be measured by methods known in the art, such as surface plasmon resonance and biolayer interferometry assay.

When we refer herein to "substantial identity" or "identity" of amino acid sequences of an antibody or antigen-binding fragment thereof, it means identity when the two amino acid sequences are optimally aligned, for example, by the programs GAP or BESTFIT using default gap weights. Non-identical amino acid residues in the compared sequences preferably differ between the sequences by conservative amino acid substitution. Amino acid sequence identity can be determined using sequence analysis software. The protein analysis software matches similar sequences using measures of similarity assigned to various substitutions, deletions, and other modifications, including conservative amino acid substitution. For example, analysis is performed using GCG software (e.g., GCG Version 6.1), FASTA (e.g., FASTA2 or FASTA3), BLASTP or TBLASTN.

When referring to "sequence identity" or "identity" in a nucleic acid sequence, it is optimally aligned and compared to another nucleic acid (or its complementary strand) with appropriate nucleotide insertion or deletion, and for example, it is measured by any well-known algorithm for sequence identity, such as FASTA, BLAST, or GAP.

As used herein, the term "subject" refers to animals infected or at risk of being infected with a virus and in need of amelioration, prevention, and/or treatment for a disease or condition such as a viral infection, preferably, mammals, more preferably humans. In certain embodiments, the subject is a human infected with or at risk of being infected with SARS-CoV-2.

The antibody and the like of the present invention specifically bind to the SARS-CoV-2 spike protein. Therefore, the antibody and the like of the present invention can also be used to detect the antigen SARS-CoV-2 virus or the SARS-CoV-2 spike protein.

"Binding affinity", which describes the binding activity of an antibody and an antigen, generally means power which is the sum of non-covalent interactions between a single binding site of a molecule (e.g., antibody) and its binding partner (e.g., antigen). "Binding affinity" is expressed as a unique numerical value that reflects a 1:1 interaction between members of a binding pair (e.g., antibody and antigen). The affinity of molecule X for its partner Y can generally be expressed by the equilibrium dissociation constant (Kd), calculated as the ratio koff/kon. See, e.g., Chen, et al. (1999) J. Mol Biol 293: 865-881. Affinity can be measured by common methods known in the art. Low-affinity antibodies generally bind an antigen slowly and tend to dissociate easily, whereas high-affinity antibodies generally bind an antigen more quickly and tend to keep the bound state longer. Examples of measurement methods include, but are not limited to, radiolabeled antigen binding assays (RIA), ELISA-based immunoassays, and surface plasmon resonance assay using Biacore (registered trademark). Methods and reagents suitable for determining the binding properties of the antibody or antigen-binding fragment thereof or altered/mutated derivative thereof of the present invention are known in the art and commercially available. In addition, instruments and software designed for such analyzes are also commercially available (e.g., Biacore (registered trademark) A100, and Biacore (registered trademark) 2000).

In certain embodiments, the antibody-antigen binding assay may be performed either as a direct binding assay or as competitive binding assay. Binding can be detected using standard ELISA or standard flow cytometric assays. In direct binding assays, a test antibody is tested for binding to its antigen, while in competitive binding assays, the ability of a test antibody to compete with known antibodies that bind to the SARS-CoV-2 spike protein is assessed. These methods are used to screen for those that provide the desired properties.

Antibodies specific for the SARS-CoV-2 spike protein can also be further labeled. Labels can be attached to the N-terminus or C-terminus of the antibody, or to the side chains of any amino acid residue. Labels are, for example, avidin-biotin, radionuclides, fluorescent dyes, or MRI-detectable labels. In certain embodiments, labeled antibodies are used in diagnostic assays, including imaging assays.

In addition to the amino acid sequences of the antibody and the like of the present invention described in the present specification, the present invention also provides nucleotide sequences that correspond to and encode the amino acid sequences. Accordingly, the present invention also includes an isolated nucleic acid having a nucleotide sequence encoding the antibody and the like of the present invention. The isolated nucleic acid is preferably cDNA. These nucleic acid sequences include nucleic acid sequences encoding part or all of the light and heavy chains and CDRs of the antibody of the present invention and these are also included in the present invention. Accordingly, the present invention also contains polynucleotide sequences encoding the VH and VL framework regions, including the CDRs and FRs, of the antibodies described in the present specification, and expression vectors for their efficient expression in cells (e.g., mammalian cells). Methods for producing antibodies using polynucleotides are known in the art, and those methods can be used as appropriate. Methods of making the antibody of the present invention using these methods are also included in the present invention.

Exemplary techniques for the production of antibodies that can be used in the present invention are described below. The antibody and the like of the present invention can be prepared using a wide range of techniques known in the art, including genetic recombination techniques, phage display techniques, or combinations thereof.

Production and screening methods for specific antibodies using gene recombination technologies are well known and commonly used techniques in the art. A DNA encoding the amino acid sequence of the antibody and the like of the present invention can be easily prepared using known techniques. For example, by referring to the sequences of SEQ ID NOs listed in Table 1, DNA sequences encoding the amino acids of each CDR can be chemically synthesized and combined arbitrarily, though the means is not limited to this. At that time, it can be combined with any DNA encoding the amino acid sequence of VH or VL, FR.

For expression of an antibody or antigen-binding fragment thereof, or a variant thereof, an expression vector containing polynucleotides encoding the antibody and the like is constructed. Thus, replicable vectors containing nucleotide sequences encoding an antibody molecule, a heavy chain or light chain of an antibody, a variable domain of a heavy or light chain of an antibody or portion thereof, or any CDR of a heavy or light chain, operably linked to a promoter, are provided, and also included in the present invention. Such vectors can contain the nucleotide sequences encoding the constant regions of the antibody molecule, and variable domains of the antibody and the like may also be cloned into vectors for expression of whole heavy chain, whole light chain, or both whole heavy and light chains. The expression vector is introduced into host cells and the transfected cells are then cultured to produce the antibody. Accordingly, the present invention includes host cells containing polynucleotides encoding amino acid sequences of the antibody and the like of the present invention. For expression of double-chain antibodies, vectors encoding both heavy and light chains may be expressed simultaneously in a host cell for expression of the entire immunoglobulin molecule.

Mammalian cell strains available as hosts for the expression of recombinant antibodies include many immortalized cell strains known in the art and available from the ATCC, including but not limited to, for example, Chinese Hamster Ovary (CHO) cells, HeLa cells, baby hamster kidney (BHK) cells, monkey kidney cells (COS), human hepatocellular carcinoma cells (e.g., HepG2), human renal epithelial cells (HREpC), human embryonic kidney cells (HEK), and numerous other cell strains. Appropriate cell strains or host systems can be chosen to ensure the correct modification and processing of the antibody or portion thereof expressed. For this reason, eukaryotic host cells that have the cellular machinery for proper primary transcript processing, glycosylation and phosphorylation of the gene product are preferably used. Such mammalian host cells include, but are not limited to, CHO, VERY, BHK, HeLa, COS, MDCK, HEK293, 3T3, W138, BT483, Hs578T, HTB2, BT2O, T47D, NS0 (mouse myeloma cell strain that does not endogenously produce any functional immunoglobulin chains), SP20, CRL7O3O and HsS78Bst cells. Further, an antibody and the like can be produced using human cell strains developed by immortalizing human lymphocytes using genetic recombination technologies, and in addition, a human cell strain PER. C6. can also be used to produce an antibody and the like.

Other cell strains that can be used as hosts for the expression of recombinant antibodies also include, for example, insect cells (e.g., Sf21/Sf9, Trichoplusiani Bti-Tn5b1-4) or yeast cells (e.g., S. cerevisiae, Pichia et al.), plant cells, and chicken cells.

The transfected cell strain is maintained in cell culture media under conditions known in the art to confer antibody expression and production. Media and culture techniques known in the art can be used without limitation. Antibody production can be carried out in large quantities by bioreactor processes using fed-batch, batch, perfusion, or continuous feed bioreactor methods known in the art, and such techniques enable the production of large quantities of antibodies.

The present invention is also a method for producing the antibody and the like of the present invention. The production method of the present invention includes a step of culturing the host cell of the present invention under conditions suitable for expression of an antibody or antigen-binding fragment thereof. Such methods may further include a step of isolating an antibody or antigen-binding fragment thereof from the host cell culture and optionally a step of blending the isolated antibody or fragment thereof into a composition.

Antibodies or antigen-binding fragments thereof or amino acid-substituted forms thereof can also be produced using a so-called phage display technology. A phage display library can be constructed with reference to the sequences described herein, and binding to the SARS-CoV-2 spike protein can be used as an index to generate an antibody or antigen-binding fragment thereof of the present invention. In the phage display method, functional antibody domains are displayed on the surface of phage particles which carry the polynucleotide sequences encoding them. A phage expressing an antigen binding domain that binds to the antigen of interest can be selected or identified using, for example, a labeled antigen or an antigen bound or captured to a solid surface or bead.

### Antibody purification and isolation

After being produced by expression using recombinant techniques, the antibody molecule may be purified by any method known in the art for purifying immunoglobulin molecules, for example, by chromatography (e.g., ion exchange chromatography, affinity chromatography, in particular by affinity to specific antigen protein A or protein G, and size exclusion column chromatography), centrifugation, difference in solubility, or any other standard protein purification techniques. Additionally, the antibody and the like of the present invention may be fused to heterologous polypeptide sequences (commonly referred to as "tags") known to facilitate purification.

Using recombinant techniques, the antibody can be produced intracellularly, in the periplasmic space, or directly secreted into a medium. If the antibody is produced intracellularly, as a first step, the particulate debris, either host cells or lysed fragments, is removed, for example, by centrifugation or ultrafiltration. If the antibody is secreted into a medium, the supernatant from the expression system is concentrated using protein concentration filters, for example, an ultrafiltration unit.

Antibody-containing preparations prepared from cells may be purified, for example, by hydroxylapatite chromatography, hydrophobic interaction chromatography, ion-exchange chromatography, gel electrophoresis, dialysis, and/or affinity chromatography, used alone or in combination with other purification steps. If the antibody contains a CH3 domain, Bakerbond ABX resin is useful for purification. Other protein purification techniques, for example, fractionation on ion exchange columns, ethanol precipitation, reverse phase HPLC, silica chromatography, heparin chromatography, anion or cation exchange resin (such as polyaspartic acid columns) sepharose chromatography, chromatofocusing, SDS-PAGE, and ammonium sulfate precipitation are also available, depending on the antibody to be recovered.

In certain embodiments, substantially purified or isolated antibodies of the present invention are provided. In certain embodiments, such purified or isolated recombinantly expressed antibodies can be administered to a patient to achieve a prophylactic or therapeutic effect.

Human antibodies can be produced using methods well known in the art. Human antibodies avoid some of the problems associated with antibodies with mouse or rat variable and/or constant regions. The presence of such mouse or rat derived proteins can cause rapid clearance of the antibodies or can cause the patient to develop an immune response against the antibodies.

Human antibodies can also be obtained by in vitro methods. For example, a phage display method, a ribosome display method and a yeast display method are mentioned. Human antibodies or antibody fragments can be made in vitro from immunoglobulin variable (V) domain gene repertoires derived from unimmunized donors by using a phage display technology (see, e.g., US Pat. No. 5,969,108). Alternatively, human antibodies may also be generated by in vitro activated B cells (see, e.g., US Pat. Nos. 5,567,610 and 5,229,275).

Immunoglobulin genes undergo various modifications during the maturation of the immune response, including recombination between V, D and J gene segments in the variable regions, isotype switching, and hypermutation. Although recombination and somatic hypermutation result in antibody diversity and affinity maturation, they can also increase the immunogenicity risk of antibodies. In general, mutations in CDR regions appear to contribute to improved affinity and function, while mutations in framework regions can increase immunogenicity risk. Thus, framework sequences can reduce risk by making them germline similar. It is desirable to remove potential structural factors that could lead to instability, aggregation, heterogeneity, or increased immunogenicity of the antibody produced. Examples of undesirable structural factors include unpaired cysteines (which can lead to unwanted disulfide binding formation or variable sulfhydryl adduct formation), N-linked glycosylation sites (structure and activity resulting in heterogeneity), as well as deamidation (e.g., NG, NS), isomerization (DG), oxidation (exposed methionine), and hydrolysis (DP) sites. Therefore, to reduce the risk of immunogenicity and improve pharmaceutical properties, it is desirable to revert framework sequences to germline, revert CDRs to germline, and/or remove structural factors. Thus, in certain embodiments, where a particular antibody differs from its respective germline sequence at the amino acid level, the antibody sequences may be backmutated to the germline sequence. Such corrective mutations can occur at one, two, three or more positions, or at any combination of mutated positions, using standard molecular biology techniques. Therefore, antibodies or antigen-binding fragments thereof having such mutable sequences can also be included in the present invention. In addition, the antibody and the like of the present invention can be subjected to mutations and amino acid substitutions from such viewpoints, and these are also included in the present invention.

In one embodiment, the antibody of the present invention is an antibody fragment or an antibody containing a fragment thereof. The antibody fragment includes a portion of a complete antibody that is the antigen-binding or variable region thereof. Examples of the antibody fragment include, but are not limited to, Fab, Fab', F(ab')2, Fd, Fv fragments, disulfide Fv, single chain antibodies (ScFv), single domain antibodies, nanobodies, and diabodies.

These fragments can be generated using techniques known in the art. For example, these can be obtained by proteolytic digestion of complete antibodies. These can also be produced directly in recombinant host cells using genetic techniques. Although not limited to this, Fab, Fv and scFv antibody fragments can all be expressed in and secreted from E. coli, thus facilitating the production of large amounts of these fragments. Alternatively, the Fab'-SH fragments can be expressed in E. coli, recovered, and chemically coupled to generate F(ab')2 fragments. F(ab')2 fragments can also be expressed in recombinant host cells and isolated from culture. Fv fragments and single chain Fv (scFv) can also be generated using other reported techniques. Fv and scFv are antibody fragments that have complete antigen-binding sites that lack constant regions. An effector protein can also be fused to either the amino- or carboxy-terminus of the scFv to create an scFv fusion protein. Various techniques are known for producing antibody fragments and can be used without limitation in the present invention.

In another embodiment, the antibody of the present invention is a domain antibody, e.g., an antibody containing small functional binding units of antibodies corresponding to the heavy chain variable (VH) or light chain variable (VL) regions of a human antibody. Examples of the domain antibody include, but are not limited to, antibodies produced using Domantis technology (e.g., WO 04/058821; WO 04/081026; WO 04/003019; WO 03/002609).

In certain embodiments, the antibody of the present invention is a linear antibody. The linear antibody contains a pair of tandem Fd segments (VH-CH1-VH-CH1) that form a pair of antigen-binding regions.

In certain embodiments, the antibody of the present invention can be monospecific, bispecific, or multispecific. The multispecific antibody is either specific for different epitopes of a single target polypeptide, or contains antigen-binding domains specific for multiple target polypeptides. Any of the multispecific antigen-binding molecules of the present invention, or variants thereof, can be constructed using molecular biology techniques known to those skilled in the art (e.g., recombinant DNA and protein expression techniques).

In certain embodiments, the SARS-CoV-2 spike protein-specific antibody that is a bispecific antibody can be made by conjugating variable regions that bind separate domains of the SARS-CoV-2 spike protein into a single binding molecule to be dual-domain specific. Properly designed and engineered bispecific antibodies can totally enhance the SARS-CoV-2 virus inhibitory activity through increased specificity and binding activity. In one example of bispecificity, a single VL segment (e.g., VL1) is combined with two different VH domains (e.g., VH1 and VH2) to provide bispecificity including two binding "arms" (VH1-VL1, and VH2-VL1). Using a single VL segment can reduce system complexity and increase efficiency in cloning, expression, and purification methods.

Other typical bispecificity formats include, but are not limited to, scFv-based bispecificity formats. The bispecificity formats of bispecific antibodies include, for example, IgG-scFv fusions, dual variable domain (DVD)-Ig, quadroma, knobs-into-holes, common light chains (e.g., common light chains having knobs-into-holes, etc.), CrossMab, CrossFab, (SEED) body, Leucine Zipper, Duobody, IgG1/IgG2, Dual Action Fab (DAF)-IgG, and Mab2 bispecificity formats. The bispecific antibodies can also be constructed using peptide/nucleic acid linkages.

The present invention also includes further modified forms of the antibody and the like of the present invention, and variants and fragments thereof, including substitutions, additions and/or deletions, and post-translational modifications of one or more amino acid residues and/or polypeptides in the variable light chain (VL) domain and/or the variable heavy chain (VH) domain and/or the Fc region. The modified forms also include antibody conjugates, in which other entities, such as proteins, peptides, drugs, or labels are covalently attached to the antibody. Such antibody alterations and modifications can be used to alter the biochemical and/or functional properties of the antibody such that they are suitable for treating and/or diagnosing SARS-CoV-2 infection. Techniques for making such alterations and modifications are known in the art and can be used without limitation in the present invention.

In certain embodiments, antibody alterations can be made by one or more amino acid alterations (e.g., substitutions, deletions and/or additions) introduced into one or more of the variable regions of the antibody. In another embodiment, amino acid alterations may be introduced into the framework regions. The altered antibodies can be screened using its activity as an index using the methods described in the present specification. Amino acid substitutions may be conservative or non-conservative as described above. Also, if desired, unnatural amino acids or amino acid analogs can be substituted or added into the antibody sequence. Examples of such amino acids include, but are not limited to, D-isomers of natural amino acids, α-aminoisobutyric acid, 4-aminobutyric acid, Abu, 2-aminobutyric acid, γ-Abu, ε-Ahx, 6-Aminohexanoic acid, Aib, 3-aminopropionic acid, ornithine, norleucine, norvaline, hydroxyproline, sarcosine, citrulline, cysteic acid, t-butylglycine, t-butylalanine, phenylglycine, cyclohexylalanine, β-alanine, fluoroamino acids, designer amino acids such as β-methyl amino acids, Cα-methyl amino acids, Nα-methyl amino acids, and other amino acid analogs.

In another embodiment, in the antibody and the like of the present invention, any cysteine residue not involved in maintaining the proper conformation of the antibody or antigen-binding fragment thereof may be replaced with, for example, serine, thereby increasing the oxidation stability of the molecule and preventing abnormal cross-linking. Also, one or more cysteine linkages can be added to the antibody to improve its stability. In particular, it is useful when the antibody or the like is an antibody fragment such as an Fv fragment.

In certain embodiments, the antibody and the like of the present invention can be bound (conjugated or covalently bonded) to other substances using methods known in the art. The substances to be bound include therapeutic agents, therapeutic adjuvants, detectable labels or solid supports. The substances to be bound to antibodies include, but are not limited to, amino acids, peptides, proteins, polysaccharides, nucleosides, nucleotides, oligonucleotides, nucleic acids, haptens, drugs, hormones, lipids, lipid aggregates, synthetic polymers, microparticles, living cells, viruses, fluorophores, chromophores, dyes, toxins, haptens, enzymes, antibodies, antibody fragments, radioisotopes, solid matrix, semi-solid matrix and combinations thereof. Methods for binding these other substances to antibodies are known in the art and can be used without limitation in the present invention.

In one embodiment, the antibody and the like of the present invention are bound to a solid support. Antibodies bound to solid supports can be used, for example, as part of screening, purification, manufacturing processes, diagnostic methods or compositions. Solid supports are generally substantially insoluble in liquid phases. Numerous supports are well known to those of skill in the art and can be used in the present invention. The solid supports include, but are not limited to, solid and semi-solid matrix such as aerogels and hydrogels, resins, beads, biochips (including thin film-coated biochips), microfluidic chips, silicon chips, multi well plates (also called microtiter plates or microplates), membranes, conductive and non-conductive metals, glass (including microscope slides) and magnetic supports. Examples of more specific solid supports include silica gel, polymeric membranes, particles, derivatized plastic films, glass beads, cotton, plastic beads, alumina gels, polysaccharides such as sepharose, polyacrylates, polystyrene, polyacrylamides, polyols, agarose, agar, cellulose, dextran, starch, FICOLL, heparin, glycogen, amylopectin, mannan, inulin, nitrocellulose, diazocellulose, polyvinyl chloride, polypropylene, polyethylene (including polyethylene glycol), nylon, latex beads, magnetic beads, paramagnetic beads, superparamagnetic beads, and the like.

In certain embodiments, the solid support may have a reactive functional group for binding to the antibody and the like of the present invention, such as a hydroxyl group, a carboxyl group, an amino group, a thiol group, an aldehyde group, a halogen group, a nitro group, a cyano group, an amide group, a urea group, a carbonate group, a carbamate group, an isocyanate group, a sulfone group, a sulfonate group, a sulfonamide group, a sulfoxide group, and the like.

In certain embodiments, the antibody of the present invention can be bound to labels for diagnostic and other measurement purposes (e.g., detection of SARS-CoV-2 virus). Labels used in binding to antibodies include, but are not limited to, chromophores, fluorophores, fluorescent proteins, phosphorescent dyes, tandem dyes, particles, haptens, enzymes and radioisotopes. Techniques for binding antibodies to these labeling substances are well known in the art and can be used in the present invention. Also, in diagnostics and other assays using labeled antibodies, detection methods based on the type of label are similarly known in the art, and such detection methods can also be used in the present invention.

The antibody and the like of the present invention can be used for treating SARS-CoV-2 virus infection, preventing SARS-CoV-2 virus infection, and/ or detecting, diagnosing and/or prognosing SARS-CoV-2 virus infection. The antibody and the like of the present invention can also be used in the treatment, prevention, or diagnosis of other coronaviruses as long as they antigen-specifically bind to those other coronaviruses.

In one embodiment, the present invention is a method of treating a subject by administering to the subject an effective amount of the antibody and the like of the present invention. In certain embodiments, substantially purified antibodies or antigen-binding fragments thereof are used. In certain embodiments, the antibody or antigen-binding fragment thereof of the present invention is administered to a subject infected or suspected of being infected with SARS-CoV-2. In certain embodiments, the antibody and the like of the present invention are administered prophylactically to subjects at risk of being infected with SARS-CoV-2. In another embodiment, the antibody and the like of the present invention are administered to a subject after infection or after the onset of symptoms, or pre-infection prophylactically. In certain embodiments, the antibody and the like of the present invention are administered to infected but asymptomatic subjects.

In certain embodiments, the antibody or antigen-binding fragment thereof of the present invention, or a pharmaceutical composition containing them, and the method of the present invention of administering them to a subject treat SARS-CoV-2 viral infection or reduce viral infections in the subject. In another embodiment, the pharmaceutical compositions of the present invention and the method of the present invention prevent SARS-CoV-2 viral infection, reduce the risk of infection, or delay infection in a subject.

The subjects to which the antibody and the like of the present invention are administered are mammals, preferably humans. In a more particular embodiment, the subject is, for example, a subject that is immunocompromised, a subject that is particularly at risk or susceptible to infection, to the SARS-CoV-2 virus. Subjects (patients) at risk include, but not limited to, those with immunocompromised conditions, the elderly (aged 65 and older), those with underlying medical conditions such as pulmonary infections, heart disease, respiratory disease, diabetes, or dialysis patients; health care workers, persons in close contact with persons with confirmed or suspected SARS-CoV-2 infection, or persons expected to come into contact with such persons.

Administration for therapy can be a single dose schedule or a multiple dose schedule, and the antibody or antigen-binding fragment thereof of the present invention can be used in passive immunization.

In certain embodiments, the antibody and the like of the present invention can be administered to a subject in combination with one or more other agents, such as antiviral agents. Other agents include, but are not limited to, for example, remdesivir, favipiravir, dexamethasone, ciclenisonide, nafamostat, camostat, ivermectin, bamilanibimab, etesevimab, casilibimab, imdevimab, and HIV protease inhibitors (e.g., lopinavir, ritonavir or combination drugs thereof, and nelfinavir). In certain embodiments, the antibody and the like of the present invention can be administered to a subject in combination with a therapeutic agent that, if any side effects associated with the antibody and the like of the present invention occur, helps combat or reduce such potential side effects. Also, therapeutic agents for diseases at risk of exacerbation or causing complications in SARS-CoV-2 infection, such as diabetes, cardiovascular disease, kidney disease, and chronic respiratory diseases such as COPD can be administered in combination to subjects. Such agents include, for example, anti-inflammatory agents (e.g., corticosteroids and non-steroidal anti-inflammatory agents), anti-infective agents, nutritional supplements such as antioxidants, and any other drugs known in the art to improve the physical or mental condition of a subject.

The present invention also provides a method for producing a pharmaceutical composition including a step of mixing the antibody and the like of the present invention with one or more pharmaceutically acceptable carriers.

The present invention also provides a pharmaceutical composition containing the antibody and the like of the present invention as active ingredients. Such pharmaceutical compositions contain a therapeutically effective amount of the antibody and the like of the present invention, and further contain a pharmaceutically acceptable carrier. As used herein, "pharmaceutically acceptable carrier" means a carrier approved for use by a regulatory agency or listed in a generally accepted pharmacopoeia for use in animals, particularly humans. The carriers include diluents, adjuvants, excipients, or vehicles with which the active ingredient is administered. Such carriers include sterile liquids such as water and oils such as those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The pharmaceutical composition, if desired, can also contain minor amounts of wetting or emulsifying agents, or pH buffering agents.

The pharmaceutical composition of the present invention can take any form, such as solutions, suspensions, emulsions, tablets, pills, capsules, powders, sustained-release formulations, and the like. Techniques for formulating pharmaceutical compositions into respective forms are known in the art, and can be referred to in the production of the pharmaceutical composition of the present invention, and formulations suitable for administration methods are carried out.

The dosage of the antibody and the like of the present invention is appropriately selected depending on the symptoms, age, condition, route of administration, and the like of the subject to be administered. When the antibody of the present invention is used for the treatment of diseases in adult patients or for the prevention of such diseases, it is usually administered, but not limited to, in a single dose at a lower limit of about 0.01 mg, preferably about 0.05 mg, and more preferably about 0.1 mg and an upper limit of about 100 mg, preferably about 50 mg, more preferably about 20 mg, and further preferably about 10 mg per kg body weight. When using an antigen-binding fragment of the antibody of the present invention, it is usual to administer a dose higher than the above dose, for example, but not limited to, about 1.5 to about 50 times, preferably about 1.5 to about 20 times, and more preferably about 1.5 to about 10 times. The frequency and interval of administration is adjusted depending on the severity of the condition. In certain embodiments, the antibody and the like of the present invention are administered as a starting dose of at least about 1 mg to about 1000 mg. In certain embodiments, the starting dose is followed by a second or multiple subsequent doses of an antibody or antigen-binding fragment thereof in an amount that is about the same as or less than that of the starting dose, separated by at least 1 to 3 days; at least 1 week; at least 2 weeks; at least 3 weeks; at least 4 weeks; at least 5 weeks; at least 6 weeks; at least 7 weeks; at least 8 weeks; at least 9 weeks; at least 10 weeks; or at least 12 weeks.

Administration and treatment plans, including dosage and administration schedule, can be determined appropriately by the physician who diagnosed the subject (patient), taking into consideration the symptoms, age, condition, route of administration, etc. of the subject, referring to the guidelines presented from various institutions as necessary.

The administration route of the pharmaceutical composition of the present invention can be arbitrarily determined in consideration of the symptom, condition and other conditions of the subject. Routes of administration include, but are not limited to, intradermal, transdermal, intramuscular, intraperitoneal, intravenous, subcutaneous, intranasal, epidural and oral routes. The pharmaceutical compositions are administered by any convenient route, such as by infusion or bolus injection, or by absorption through epithelial or mucocutaneous linings (e.g., oral mucosa, rectal, and intestinal mucosa, etc.), and may be administered with other biologically active agents. Administration can be systemic or local.

Various delivery systems are known for administration of active pharmaceutical ingredients, and these can be used to administer the pharmaceutical composition of the present invention. Examples thereof include encapsulation in liposomes, microparticles, nanoparticles, and microcapsules. Also, in certain embodiments, the pharmaceutical compositions can be delivered in a controlled release system. The controlled-release system places the composition near the target, thus requiring only a small systemic dose.

When administering the pharmaceutical composition of the present invention by injection, injectable preparations include intravenous, subcutaneous, intradermal, intracranial, intraperitoneal and intramuscular injection forms, or drip infusion forms and the like. These injectable preparations can be prepared with reference to known methods. Injectable preparations can be prepared, for example, by dissolving, suspending, or emulsifying the antibody and the like of the present invention, or a salt thereof in a sterile aqueous or oily medium commonly used for injection. Aqueous vehicles for injection include, for example, physiological saline, isotonic solutions containing glucose, and other adjuvants, and can be used in combination with a suitable solubilizer such as alcohols (e.g., ethanol), polyhydric alcohols (e.g., propylene glycol, polyethylene glycols), nonionic surfactants [e.g., polysorbate 80, HCO-50 (polyoxyethylene (50 mol) adduct of hydrogenated castor oil)], and the like. As an oily medium, for example, sesame oil, soybean oil and the like are used, and they can be used in combination with a solubilizer such as benzyl benzoate, benzyl alcohol and the like. Injections prepared are also preferably filled into suitable ampoules.

When the pharmaceutical composition of the present invention is an injection, it can be prepared as a ready-to-use injection or as a prefilled syringe. The pharmaceutical compositions of the present invention are administered subcutaneously or intravenously with standard needles and syringes. A pen delivery device can also be used for subcutaneous administration. Pen delivery devices are either reusable or disposable.

The pharmaceutical compositions, whether oral or parenteral, are prepared in dosage forms prepared in unit doses to suit the desired dosage of the active ingredient. Dosage forms containing unit doses include, for example, tablets, pills, capsules, injections (ampoules), suppositories, and the like. The amount of the antibody to be included is generally preferably from about 5 to about 1000 mg of the antibody and the like per dosage form in a unit dose.

The pharmaceutical compositions containing the antibody and the like of the present invention are useful for treating and/or preventing SARS coronavirus infection, particularly diseases, disorders, or conditions associated with SARS coronavirus such as SARS-CoV-2 infection. The pharmaceutical compositions of the present invention are also useful for ameliorating at least one symptom associated with SARS coronavirus infection, particularly SARS-CoV-2 virus infection. Such symptoms include, but are not limited to, inflammation in lungs, alveolar damage, fever, respiratory disorders (nasal congestion, runny nose, sore throat, cough, shortness of breath), headache, malaise, dysgeusia, olfactory disturbances, diarrhea, vomiting, hypercoagulability, thrombosis, Kawasaki-like vasculitis, renal dysfunction (e.g., nephritis), organ failure, pneumonia, septic shock and death. In certain embodiments, the antibody of the present invention is useful for treating subjects suffering from severe and acute respiratory infections caused by the SARS-CoV-2 virus. In certain embodiments, the antibody of the present invention is useful in reducing viral titers in a host. In one embodiment, the antibody of the present invention is useful in preventing or reducing pulmonary inflammation in subjects infected with SARS-CoV-2. In one embodiment, the antibody of the present invention is useful in preventing or reducing interstitial, peribronchiolar or perivascular inflammation, alveolar damage, and pleural changes in subjects infected with SARS-CoV-2.

The antibody and the like of the present invention can be used in combination with other drugs, or as a combination drug with other drugs. Alternatively, two or more of the antibodies and the like of the present invention can be used in combination, and in such a case, it is preferable to combine antibodies or antigen-binding fragments thereof that recognize different epitopes. The second therapeutic agent that can be used in combination with the antibody and the like of the present invention or combined with the antibody and the like of the present invention includes those described above. A synergistic effect is expected by these combined use or combinations. It is also possible to use the antibody or antigen-binding fragment thereof of the present invention in combination with other anti-SARS-CoV-2 antibodies ("cocktail therapy").

The diagnostic method using the antibody and the like of the present invention includes a step of contacting a sample collected from a subject with the antibody and the like. Such a sample is not particularly limited as long as it is a sample that is collected from a subject and is suspected to contain a virus or part of a virus, and includes samples taken from nasal passage, sinuses, salivary gland, lung, liver, pancreas, kidney, ear, eye, placenta, gastrointestinal tract, heart, ovary, pituitary gland, adrenal gland, thyroid, brain, skin, or blood vessels (preferably peripheral vessels). Typical examples include saliva, blood, and nasal/pharyngeal swabs.

Anti-SARS-CoV-2(S) antibodies of the present invention can be used to detect or measure SARS-CoV in a sample, e.g., for diagnostic purposes. In certain embodiments, one or more antibody and the like of the present invention can be used in assays to detect diseases or disorders, such as viral infections. A typical diagnostic assay for SARS-CoV-2 includes, for example, a step of bringing a sample (preferably saliva, blood, or nasal/pharyngeal swab) obtained from a patient into contact with the anti-SARS-CoV-2(S) antibody of the present invention, wherein the anti-SARS-CoV-2(S) antibody is labeled with a detectable label or reporter molecule or used as a capture ligand for selectively isolating SARS-CoV-2 from a patient sample. Alternatively, an unlabeled anti-SARS-CoV-2(S) antibody can be used in combination with a secondary antibody that itself is detectably labeled. The detectable labels or reporter molecules are radioisotopes; fluorescent or chemiluminescent moieties such as fluorescein isothiocyanate, or rhodamine; or enzymes such as alkaline phosphatase, β-galactosidase, horseradish peroxidase, or luciferase. Assay methods used to detect or measure SARS-CoV-2 in a sample include, for example, enzyme-linked immunosorbent assay (ELISA), radioimmunoassay (RIA), and fluorescence-activated cell sorting (FACS). Diagnostic and detection methods using antibodies including these are known in the art, and such known methods can be used in the present invention.

The antibody and the like of the present invention can also be used as a diagnostic kit for SARS-CoV-2 virus infection. Furthermore, since the antibody and the like of the present invention specifically bind to the RBD of the SARS-CoV-2 spike protein, it can also be used for quality control of vaccine production.

### [Examples]

The present invention will be specifically described below with reference to examples, but the present invention is not limited to the following examples.

This research plan has been ethically reviewed and approved by the Factory of Life Sciences, Kumamoto University and Kumamoto City Hospital (Ethics No. 2013). Covid-19 convalescent cases were selected, informed consent was obtained, and blood specimens were collected by doctors in charge at Kumamoto City Hospital (Hajime Iwagoe, Chief, et al.), who provided them to the present inventors for investigation.

### (Example 1) Screening for neutralizing monoclonal antibodies

Two cases considered to have strong neutralizing activity in plasma were selected among COVID19 infection convalescent cases, and the following screening was performed. B cells were isolated from plasma of two selected cases, and antibodies were recovered. In summary, we proceeded as follows. With reference to the report of Boehmer et al. (Nature protocols. Published online 15 September 2016; doi: 10.1038/nprot.2016), from peripheral blood mononuclear cells isolated from blood samples, 7AAD⁻, CD19⁺, IgG⁺, IgM⁻, CD27⁺ B cells were subjected to single cell sorting using FACS AriaIII. With reference to the report of Tiller et al. (J Immunol Methods. 329: 112-124, 2008), the antibody was recovered as follows. mRNA was recovered from the obtained cells, the IgG variable region was amplified from the recovered mRNA by RT-PCR, and paired genes of heavy and light chains were selected. Each heavy chain and light chain gene was inserted into an expression vector, transfected into HEK293T cells to express the antibody, and the recombinant antibody (recombinant IgG; rIgG) produced in the supernatant was collected. A total of 1102 clones were collected by this method.

### (Example 2) Screening for SARS-CoV-2 spike protein-binding antibodies

Using a total of 1102 clones obtained in Example 1, antibodies that bind to the SARS-CoV-2 spike protein were screened as follows.

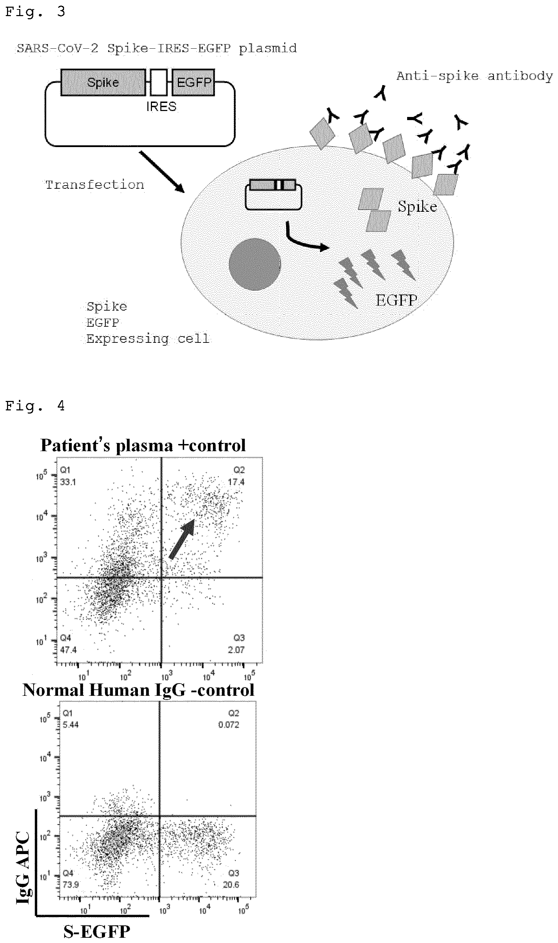

As shown in FIG. 3, plasmids co-expressing the SARS-CoV-2 spike protein and EGFP were transfected into HEK293T cells to express the spike protein on the cell surface. Two days after transfection, the cells were detached with trypsin, suspended in 0.2% BSA-PBS, and used as spike protein and EGFP-expressing cells. For 50 µL of the spike protein and EGFP-expressing cell suspension, the cell culture supernatant (50 µL) of the clone obtained in Example 1 assumed to contain anti-spike antibody, or control plasma (infected plasma and normal plasma) (diluted 1: 500, 50 µL) sample was mixed and allowed to stand at room temperature for 15 minutes. The cells were separated by centrifugation, the supernatant was discarded, the cells were suspended with 0.2% BSA-PBS, washed, and then suspended with 50 µL of 200-fold diluted APC-conjugated anti-human IgG (goat antibody) antibody, and it was allowed to stand at room temperature for 15 minutes. After washing the cells with 0.2% BSA-PBS, they were fixed with 4% paraformaldehyde-PBS. The stained cells were analyzed by flow cytometry, EGFP-positive cells were gated as spike protein-expressing cells, and the spike protein-binding activity of the antibody was determined by the APC-positive rate of the EGFP⁺ cell population. FIG. 4 shows the results of analysis by flow cytometry using patient plasma, and arrows indicate spike protein binding activity. As a result of flow cytometric analysis, a total of 88 clones expressing spike protein binding antibodies were screened from a total of 1102 clones.

### (Example 3) Screening for RBD binding antibodies

Using the 88 clones screened in Example 2, antibodies that bind to the spike protein receptor binding domain (RBD) were screened as follows.

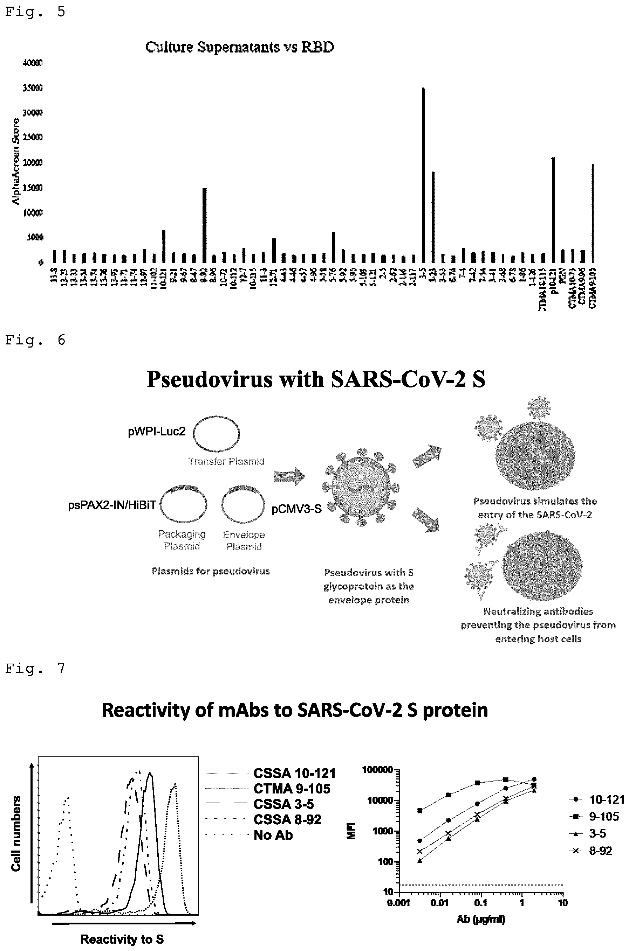

An AlphaScreen system based on the LOCI method, in which two types of fluorescently labeled beads are used to label an antigen and an antibody and the antigen-antibody binding reaction can be measured using the correlation between the distance between the beads and the emission intensity, was used to assess the binding of the cloned rIgG to the spike protein receptor binding domain (RBD) of SARS-CoV-2. The binding between biotinylated RBD (with His tag) provided by Professor Takao Hashiguchi of Kyushu University and rIgG was measured by AlphaScreen score using anti-6xHis acceptor beads and protein A donor beads. Then, 5 µL culture supernatant of each clone containing antibody, 5 µL biotinylated RBD (20 µg/mL in PBS), 5 µL acceptor beads (5 mg/mL), and 5 µL protein A donor beads (5 mg/mL) were mixed to prepare a reaction solution (20 µL), and reacted. In 11 clones out of 88 anti-spike protein antibody clones, an increase in score was observed compared to the reaction between polyglobulin-N and RBD (mean: 13,372 vs. 2,723). FIG. 5 shows part of the binding activity data using the AlphaScreen system. As shown in FIG. 5, clones CSSA10-121, CSSA8-92, CSSA12-71, CSSA5-76, CSSA3-5, CSSA3-23, and CTMA9-105 exhibited significant RBD binding activity.

The table below summarizes the number of clones screened from two selected cases (CSSA 0504 and CTMA 0430) in Examples 1 to 3.

| Date | Donor ID | | clones | S binding abs | RBD binding abs |
|---|---|---|---|---|---|
| 200504 | CSSA 0504 | | 658 | 55 | 6 |
| 200430 | CTMA0430 | | 444 | 33 | 5 |
| | | total | 1102 | 88 | 11 |

As described above, these studies yielded a total of 1102 rIgGs from two cases, CTMA and CSSA, of which 88 antibodies bound to the SARS-CoV-2 spike protein. Further, spike protein RBD-binding activity was confirmed in 11 clones out of 88 antibodies.

### (Example 4) Screening for antibodies with neutralizing activity

Plasmids expressing antibody heavy and light chains were transfected into HEK293T cells, and the SARS-CoV-2 neutralizing activity of the recombinant antibody contained in the cell culture supernatant was examined in a pseudovirus neutralization test. An outline is shown in FIG. 6. Pseudoviruses were made by transfecting into HEK293T cells with packaging plasmid psPAX2-IN/HiBiT, transfer plasmid pWPI-Luc2, and SARS-CoV-2 Spike expression plasmid pCMV3-SARS2-S at a ratio of 2: 2: 1. Two days after transfection, the culture supernatant was collected, passed through a 0.22 µM filter, stored at -80°C, and used for the following tests. For the neutralization test, 293FT-DSP1-7-ACE2-TMPRSS2 cells (provided by Professor Inoue, The Institute of Medical Science, The University of Tokyo) were used, and the day before, 1×10⁵ cells/mL cell suspension was seeded at 100 µL/well in a 96-well plate. To another 96-well plate, the cell culture supernatant of each clone containing the recombinant antibody was diluted twice with culture medium and added at 110 µL/well, and then 200 TCID₅₀ of pseudovirus was added at 110 µL/well and cultured at 37°C for 1 hour (hereinafter referred to as "mixture of culture supernatant and virus"). The experiment was performed with 2 wells for each sample. The cell culture supernatant prepared the day before was discarded, and a mixture of the culture supernatant and virus was added and cultured. After 5 hours, the mixture of the culture supernatant and virus was discarded, 200 µL of fresh culture medium was added, and the cells were further cultured. Two days after infection, the cell supernatant was discarded, 30 µL of lysis buffer was added, and the cells were lysed by stirring for 10 minutes. 10 µL of the cell lysate was transferred to a white 96-well plate, 50 µL of luciferase substrate was added, and fluorescence was measured. The RLU (relative light unit) of the virus-only sample was defined as 100% infection, and the RLU of the virus-free sample was defined as 0% infection, and the neutralizing activity by the cell culture supernatant of each clone was determined by how much RLU was reduced from 100% infection. The neutralizing activity of the cell culture supernatant of the clones that showed SARS-CoV-2 spike protein-binding activity was measured, and the clones that showed 50% or more of the neutralizing activity were selected as neutralizing antibodies. Six clones (6-74, 3-5, 5-92, 8-92, 10-121, and 9-105) were selected from the 73 clones that could be measured.

### (Example 5) Neutralization test for purified antibody prepared

A purified antibody was prepared from each selected clone and a neutralization test was performed using the purified antibody. Preparation of purified antibody from cell culture supernatant was performed according to a conventional method. The neutralization test with the purified antibody was performed in the same manner as the neutralization test using the culture supernatant, but the purified antibody was serially diluted 5-fold with 3 wells of each sample. The IC₅₀ (50% inhibitory concentration) of the antibody was calculated and used as an index of neutralizing activity. As a result, strong neutralizing activity was detected in the following four clones among the measured clones.

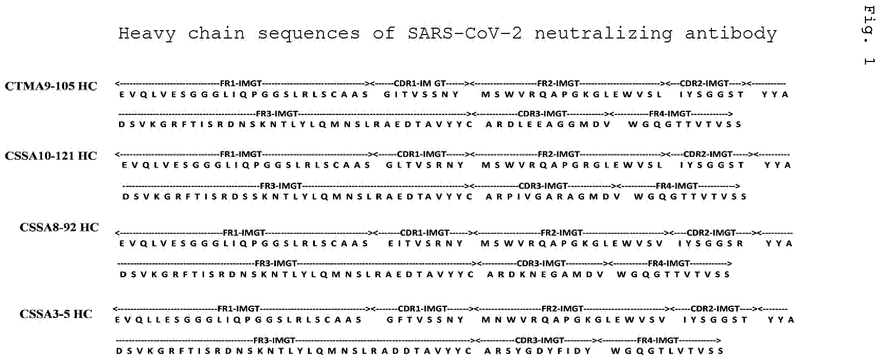

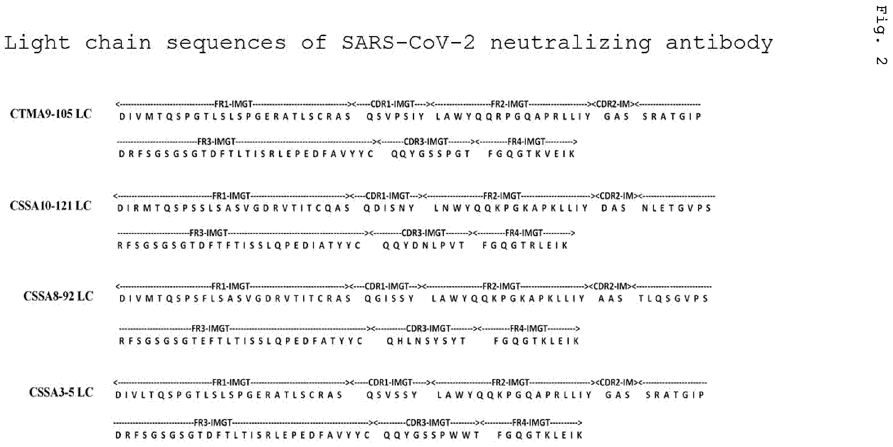

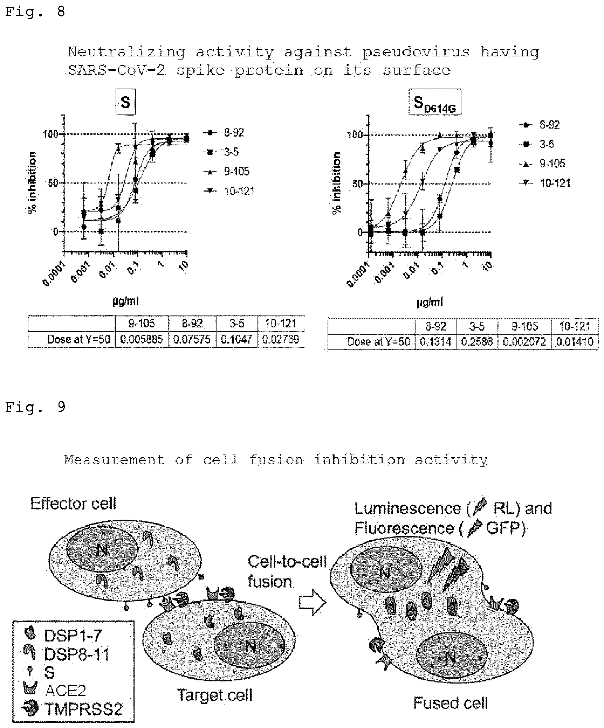

From the results of the above binding test and neutralization test, among the 11 clones that bind to RBD, among the clones whose neutralizing activity was detedtec, CTMA 9-105, CSSA 10-121, CSSA 8-92, and CSSA 3-5 four antibodies were confirmed to exhibit strong neutralizing activity. The nucleotide sequences of the heavy and light chain variable regions of these anti-spike protein antibodies were analyzed using the international immunogenetics information system (IMGT), and it was confirmed that each clone was distinct. The amino acid sequences of the heavy chain variable regions and light chain variable regions of the 4 antibodies with potent neutralizing ability are shown in FIGs. 1 and 2, respectively. In addition, these four antibodies all showed strong binding activity to the spike protein in the spike protein binding test using the purified antibody, and CTMA9-105 in particular showed strong binding activity at low concentrations. The results are shown in FIG. 7. Furthermore, FIG. 8 shows the results of the pseudovirus neutralization test using the purified antibodies of the 4 antibodies. All four antibodies showed strong neutralizing activity against two kinds of spike viruses (Wuhan type S, European type S_{D614G}). Among them, CTMA9-105 exhibited surprisingly strong activity with an IC₅₀ of 0.002-0.006 µg/mL.

### (Example 6) Cell fusion inhibition test

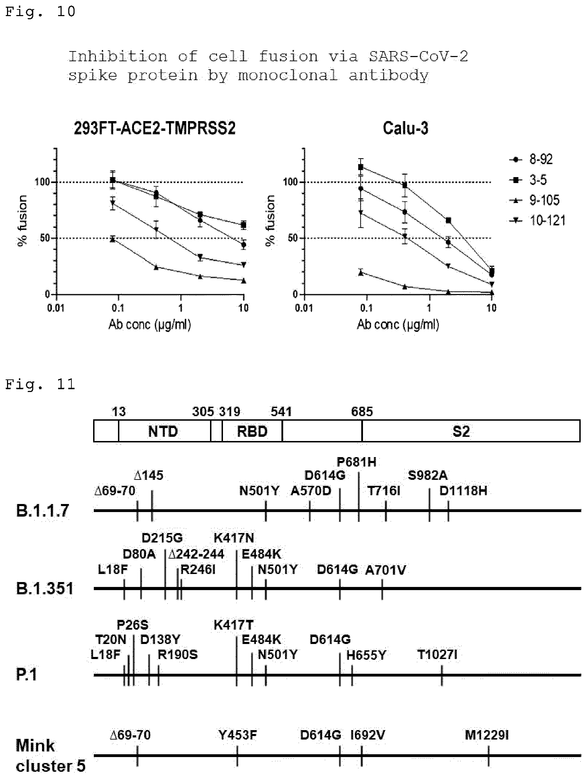

As shown in FIG. 9, the cell fusion activity was measured in which a DSP (dual split protein) based on GFP and luciferase protein is used, and when cell fusion occurs, the DSP of each cell binds and exhibits activity as an index. Effector cells expressing the SARS-CoV-2 spike protein and DSP8-11 (provided by Professor Inoue, The Institute of Medical Science, The University of Tokyo) were prepared at 3×10³ cells/ml and seeded on a 96-well plate at 100 µL/well. 293FT-DSP1-7-ACE2-TMPRSS2 or Calu-3-DSP1-7 used as target cells (provided by Professor Inoue, The Institute of Medical Science, The University of Tokyo) was prepared at 3×10³ cells/mL, and seeded at 3 mL/well on a 6-well plate. After overnight culture, target cell plates were washed with fresh medium and cultured with luciferase substrate in medium for 2-4 hours. The effector cells were washed with fresh culture medium, and 50 µL of samples such as cell culture supernatant and purified antibody were added and cultured for 1 hour. The target cell suspension was then added to the effector cells at 50 µL/well. Fluorescence was measured over time and cell fusion was detected by an increase in RLU. The RLU of the sample-free medium alone was defined as 100% cell fusion, and the cell fusion inhibitory activity of the sample was determined by how much RLU was reduced from 100%. Using two types of target cells, the inhibitory activity of SARS-CoV-2 spike protein-mediated cell fusion was measured, and the results are shown in FIG. 10. All four of the above antibodies showed potent activity. Among them, 9-105 inhibited cell fusion at low concentrations.

### (Example 7) Preparation of SARS-CoV-2 mutant strain pseudovirus

Expression plasmids expressing spike proteins of SARS-CoV-2 mutant strains, B.1.1.7, B1.351, P.1, and Mink cluster 5 were prepared by mutation insertion by PCR based on the wild-type Spike (614G) expression plasmid. FIG. 11 shows the amino acid mutations in the Spike protein of each mutant strain. The spike expression plasmid of the SARS-CoV-2 mutant strain, and the lentiviral packaging plasmid psPAX2-IN/HiBiT, transfer plasmid pWPI-Luc2 were together transfected into 293 T cells, and the supernatant after two days was harvested and frozen, and used as pseudoviruses.

### (Example 8) Measurement of neutralizing activity (1)

Five monoclonal antibodies screened (6-74, 3-5, 8-92, 10-121, and 9-105) were used to measure neutralizing activity. 200 TCID₅₀ (100 µL) of the pseudovirus was added to the diluted antibody (100 µE), incubated for 1 hour, and added to 293FT/DSP/ACE2/TMPRSS2 cells in a 96 well plate. After 2 hours of incubation, the mixed solution of antibody and virus was discarded and fresh medium was added. After 48 hours of incubation, luciferase activity was measured with a luciferase assay system (Promega) and an EnSpire Multimode Plate Reader (PerkinElmer).

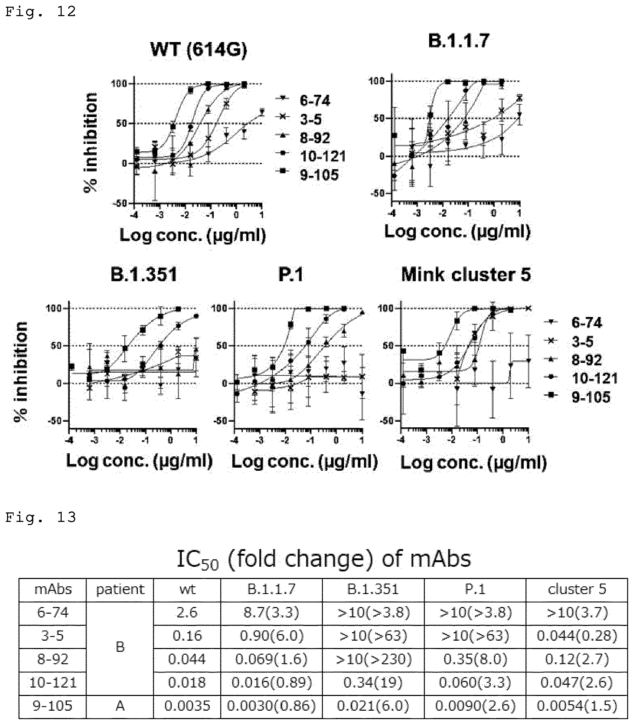

The measured neutralization data are shown in FIG. 12. FIG. 13 shows the calculated results of IC₅₀.

From the above results, among the mutant strains analyzed here, the Great Britain-derived mutant strain B.1.1.7 strain and the Denmark-derived mink cluster 5 strain showed almost the same neutralization sensitivity as the wild type, and showed resistance against antibodies with weak neutralizing activity such as monoclonal antibodies 6-74 and 3-5, however, it was revealed that monoclonal antibodies 9-105, 120-121, and 8-92 with strong neutralizing activity were effective against these mutant strains as well as the wild-type. On the other hand, the South Africa-derived B. 1.351 strain and the Brazil-derived P.1 strain acquired neutralization resistance, however, the monoclonal antibodies 9-105 and 10-121 were able to neutralize both mutant strains. In particular, the monoclonal antibody 9-105 maintained very high neutralizing activity. Mutations of K417N/T, E484K, and N501Y in the RBD region are considered to be largely involved in the neutralization resistance of the mutant strain against RBD antibody. Since N501Y exists in the B.1.1.7 strain not showing neutralization resistance, contribution to neutralization resistance is considered to be limited. K417N and E484K have been reported to be neutralizing escape mutations against RBD antibody, and this is presumed to be the reason why B. 1.351 strain having these mutations became neutralization resistant. The P.1 strain has the E484K mutation, but the fact that it has the K417T mutation instead of the K417N mutation may have contributed to the maintenance of neutralization sensitivity.

Of the 4 neutralizing antibodies screened, 2 antibodies with strong neutralizing activity (9-105 and 10-121) showed effective neutralizing activity against the 4 mutant strains. In particular, the monoclonal antibody 9-105 maintained a very strong neutralizing activity with an IC₅₀ value of 0.021 µg/mL against the South African mutant strain (B.1.351), which showed the most neutralization resistance.

As shown in the figure, the neutralizing activity (IC₅₀ value) against SARS-CoV-2 (wt) of two antibodies (9-105 and 10-121) with strong neutralizing activity was 0.0035 µg/mL and 0.018 µg/mL, respectively, but when measured using human lung cancer cells (alveolar epithelial cells, Calu-3) and gastrointestinal mucosal epithelial cells (Caco-2) instead of 293F/ACE2/TMPRSS2 cells, strong neutralizing activity was similarly confirmed.

**Table 3**

| mAb | 293F/ACE2 /TMPRSS2 | Calu-3 | Caco-2 |
|---|---|---|---|
| 9-105 | 0.0035 *µ*g/mL | 0.0019 *µ*g/mL | 0.00092 *µ*g/mL |
| 10-121 | 0.018 *µ*g/mL | 0.12 *µ*g/mL | 0.015 *µ*g/mL |

### (Example 9) Preparation of other SARS-CoV-2 viral stock

Indian mutant strains (B.1.617.1 and B.1.617.2) which are SARS-CoV-2 mutant strains isolated at the Tokyo Metropolitan Institute of Public Health were infected into Vero cells, and three days later, cell supernatants were harvested and stored frozen as a virus stock.

### (Example 10) Measurement of neutralizing activity (2)

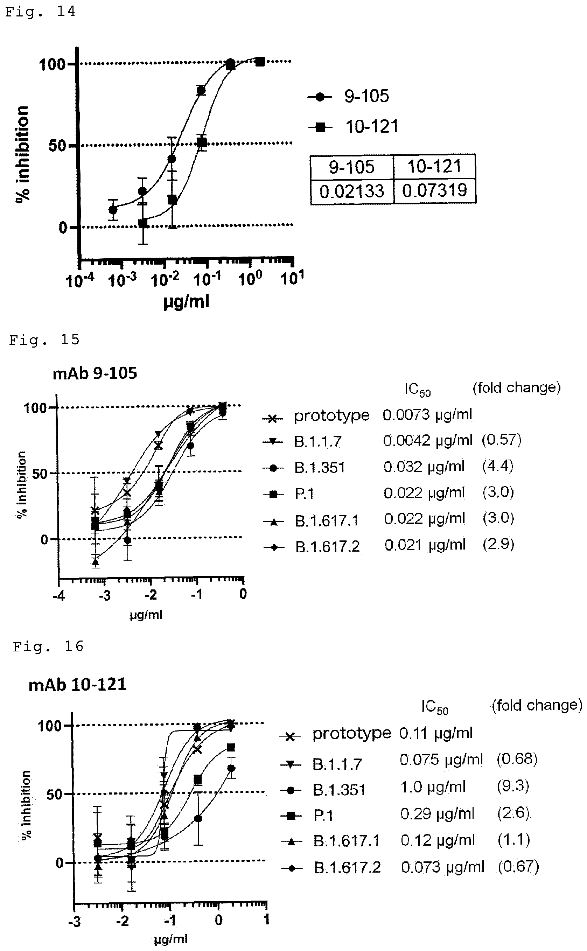

Using the two viruses (B.1.617.1 and B.1.617.2) prepared in Example 9, the neutralizing activity of the monoclonal antibodies (10-121 and 9-105) against the Indian mutant strain was measured by the plaque method. Neutralization data measured with Delta strain (1.617.2) and calculated IC₅₀ (µg/ml) results are shown in FIG. 14. Monoclonal antibodies 10-121 and 9-105 were able to neutralize Delta strain (1.617.2).

Similarly, the neutralizing activity against the Indian mutant strain was compared with that against the other mutant strains. The measured neutralization data and calculated IC₅₀ results are shown in FIGs 15 and 16. The monoclonal antibody 9-105 showed very high neutralizing activity against all mutant strains.

The foregoing merely illustrates objects and subjects of the present invention and is not intended to be limiting the accompanying Claims. Without departing from the accompanying Claims, various modifications and alterations to the described embodiments will be apparent to those skilled in the art in view of the teachings herein.

### [Industrial Applicability]

The present invention provides antibodies against SARS-CoV-2. The antibody of the present invention can be produced in large quantities and is useful as a therapeutic agent for coronavirus infection.

## Claims

1. An antibody or antigen binding fragment thereof that binds to the Receptor Binding Domain (RBD) present in the spike protein of the coronavirus (SARS-CoV-2) and is capable of neutralizing the SARS-CoV-2 virus,
comprising the following heavy chains CDR1-3 and light chains CDR1-3:
(1) A heavy chain CDR1 selected from the group consisting of a heavy chain CDR1 of SEQ ID NO: 9 (GITVSSNY), a heavy chain CDR1 of SEQ ID NO: 12 (GLTVSRNY), a heavy chain CDR1 of SEQ ID NO: 14 (EITVSRNY), a heavy chain CDR1 of SEQ ID NO: 17 (GFTVSSNY), a heavy chain CDR1 consisting of a sequence in which one or two amino acids are deleted, substituted or added in any of heavy chains CDR1 selected from the group consisting of SEQ ID NOs: 9, 12, 14 and 17, and a heavy chain CDR1 having 90% or more substantial identity with any of heavy chains CDR1 selected from the group consisting of SEQ ID NOs: 9, 12, 14 and 17,
(2) A heavy chain CDR2 selected from the group consisting of a heavy chain CDR2 of SEQ ID NO: 10 (IYSGGST), a heavy chain CDR2 of SEQ ID NO: 15 (IYSGGSR), a heavy chain CDR2 consisting of a sequence in which one or two amino acids are deleted, substituted or added in any of heavy chains CDR2 selected from the group consisting of SEQ ID NOs: 10 and 15, and a heavy chain CDR2 having 90% or more substantial identity with any of heavy chains CDR2 selected from the group consisting of SEQ ID NOs: 10 and 15,
(3) A heavy chain CDR3 selected from the group consisting of a heavy chain CDR3 of SEQ ID NO: 11 (ARDLEEAGGMDV), a heavy chain CDR3 of SEQ ID NO: 13 (ARPIVGARAGMDV), a heavy chain CDR3 of SEQ ID NO: 16 (ARDKNEGAMDV), a heavy chain CDR3 of SEQ ID NO: 18 (ARSYGDYFIDY), a heavy chain CDR3 consisting of a sequence in which one, two or three amino acids are deleted, substituted or added in any of heavy chains CDR3 selected from the group consisting of SEQ ID NOs: 11, 13, 16 and 18, and a heavy chain CDR3 having 90% or more substantial identity with any of heavy chains CDR3 selected from the group consisting of SEQ ID NOs: 11, 13, 16 and 18,
(4) A light chain CDR1 selected from the group consisting of a light chain CDR1 of SEQ ID NO: 19 (QSVPSIY), a light chain CDR1 of SEQ ID NO: 22 (QDISNY), a light chain CDR1 of SEQ ID NO: 25 (QGISSY), a light chain CDR1 of SEQ ID NO: 28 (QSVSSY), a light chain CDR1 consisting of a sequence in which one or two amino acids are deleted, substituted or added in any of light chains CDR1 selected from the group consisting of SEQ ID NOs: 19, 22, 25 and 28, and a light chain CDR1 having 90% or more substantial identity with any of light chains CDR1 selected from the group consisting of SEQ ID NOs: 19, 22, 25 and 28,
(5) A light chain CDR2 selected from the group consisting of a light chain CDR2 of SEQ ID NO: 20 (GAS), a light chain CDR2 of SEQ ID NO: 23 (DAS), a light chain CDR2 of SEQ ID NO: 26 (AAS), and a light chain CDR2 consisting of a sequence in which one amino acid is deleted, substituted or added in any of light chains CDR2 selected from the group consisting of SEQ ID NOs: 20, 23 and 26, and
(6) A light chain CDR3 selected from the group consisting of a light chain CDR3 of SEQ ID NO: 21 (QQYGSSPGT), a light chain CDR3 of SEQ ID NO: 24 (QQYDNLPVT), a light chain CDR3 of SEQ ID NO: 27 (QHLNSYSYT), a light chain CDR3 of SEQ ID NO: 29 (QQYGSSPWWT), a light chain CDR3 consisting of a sequence in which one or two amino acids are deleted, substituted or added in any of light chains CDR3 selected from the group consisting of SEQ ID NOs: 21, 24, 27 and 29, and a light chain CDR3 having 90% or more substantial identity with any of light chains CDR3 selected from the group consisting of SEQ ID NOs: 21, 24, 27 and 29.

2. The antibody or antigen-binding fragment thereof according to claim 1, wherein the heavy chain CDR1 is a heavy chain CDR1 selected from the group consisting of SEQ ID NOs: 9, 12, 14 and 17, the heavy chain CDR2 is a heavy chain CDR2 selected from the group consisting of SEQ ID NOs: 10 and 15, the heavy chain CDR3 is a heavy chain CDR3 selected from the group consisting of SEQ ID NOs: 11, 13, 16 and 18, the light chain CDR1 is a light chain CDR1 selected from the group consisting of SEQ ID NOs: 19, 22, 25 and 28, the light chain CDR2 is a light chain CDR2 selected from the group consisting of SEQ ID NOs: 20, 23 and 26, and the light chain CDR3 is a light chain CDR3 selected from the group consisting of SEQ ID NOs: 21, 24, 27 and 29.

3. The antibody or antigen-binding fragment thereof according to claim 1, wherein the heavy chain CDR1 is a heavy chain CDR1 of SEQ ID NO: 9, the heavy chain CDR2 is a heavy chain CDR2 of SEQ ID NO: 10, the heavy chain CDR3 is a heavy chain CDR3 of SEQ ID NO: 11, the light chain CDR1 is a light chain CDR1 of SEQ ID NO: 19, the light chain CDR2 is a light chain CDR2 of SEQ ID NO: 20, and the light chain CDR3 is a light chain CDR3 of SEQ ID NO: 21.

4. The antibody or antigen-binding fragment thereof according to claim 1, wherein the heavy chain CDR1 is a heavy chain CDR1 of SEQ ID NO: 12, the heavy chain CDR2 is a heavy chain CDR2 of SEQ ID NO: 10, the heavy chain CDR3 is a heavy chain CDR3 of SEQ ID NO: 13, the light chain CDR1 is a light chain CDR1 of SEQ ID NO: 22, the light chain CDR2 is a light chain CDR2 of SEQ ID NO: 23, and the light chain CDR3 is a light chain CDR3 of SEQ ID NO: 24.

5. The antibody or antigen-binding fragment thereof according to claim 1, wherein the heavy chain CDR1 is a heavy chain CDR1 of SEQ ID NO: 14, the heavy chain CDR2 is a heavy chain CDR2 of SEQ ID NO: 15, the heavy chain CDR3 is a heavy chain CDR3 of SEQ ID NO: 16, the light chain CDR1 is a light chain CDR1 of SEQ ID NO: 25, the light chain CDR2 is a light chain CDR2 of SEQ ID NO: 26, and the light chain CDR3 is a light chain CDR3 of SEQ ID NO: 27.

6. The antibody or antigen-binding fragment thereof according to claim 1, wherein the heavy chain CDR1 is a heavy chain CDR1 of SEQ ID NO: 17, the heavy chain CDR2 is a heavy chain CDR2 of SEQ ID NO: 10, the heavy chain CDR3 is a heavy chain CDR3 of SEQ ID NO: 18, the light chain CDR1 is a light chain CDR1 of SEQ ID NO: 28, the light chain CDR2 is a light chain CDR2 of SEQ ID NO: 20, and the light chain CDR3 is a light chain CDR3 of SEQ ID NO: 29.

7. The antibody or antigen-binding fragment thereof according to claim 1, which has a heavy chain variable region set forth in SEQ ID NO: 1 and a light chain variable region set forth in SEQ ID NO: 5.

8. The antibody or antigen-binding fragment thereof according to claim 1, which has a heavy chain variable region set forth in SEQ ID NO: 2 and a light chain variable region set forth in SEQ ID NO: 6.

9. The antibody or antigen-binding fragment thereof according to claim 1, which has the heavy chain variable region set forth in SEQ ID NO: 3 and the light chain variable region set forth in SEQ ID NO: 7.

10. The antibody or antigen-binding fragment thereof according to claim 1, which has a heavy chain variable region as set forth in SEQ ID NO: 4 and a light chain variable region as set forth in SEQ ID NO: 8.

11. The antibody or antigen-binding fragment thereof according to any one of claims 1 to 10, wherein in a neutralization assay using pseudoviruses, the antibody activity in neutralizing the SARS-CoV-2 virus has a high neutralizing ability expressed as 50% inhibitory concentration (IC₅₀ µg/mL) in a range from about 0.001 µg/mL to about 1 µg/mL.

12. The antibody or antigen-binding fragment thereof according to any one of claims 1 to 10, showing neutralizing activity against at least one SARS-CoV-2 mutant strain selected from the group consisting of B.1.1.7 mutant strain, mink cluster 5 mutant strain, B.1.351 mutant strain, P.1 mutant strain, P.3 mutant strains, B.1.617 mutant strain, R.1 mutant strain, and B.1.427/B.1.429 mutant strains.

13. The antibody or antigen-binding fragment thereof according to any one of claims 1 to 10, showing neutralizing activity against a SARS-CoV-2 mutant strain having one or more mutations selected from the group consisting of K417T/N mutation, E484K mutation, N501Y mutation, and L452R mutation.

14. The antibody or antigen-binding fragment thereof according to any one of claims 1 to 10, selected from the group consisting of immunoglobulin molecules, monoclonal antibodies, human antibodies, chimeric antibodies, CDR-grafted antibodies, Fab, Fab', F(ab')2, Fd, Fv, disulfide-bound Fv, scFv, single domain antibodies, nanobody antibodies, diabody antibodies, bispecific antibodies, and multi-specific antibodies.

15. A nucleic acid encoding the antibody or antigen-binding fragment thereof according to any one of claims 1-14.

16. A vector comprising the nucleic acid according to claim 15.

17. A host cell comprising the nucleic acid according to claim 15 or the vector according to claim 16.

18. A method for producing the antibody or antigen-binding fragment thereof according to any one of claims 1 to 14, comprising a step of culturing the host cell according to claim 15 under conditions suitable for expressing the antibody or antigen-binding fragment thereof.

19. A pharmaceutical composition comprising the antibody or antigen-binding fragment thereof according to any one of claims 1 to 14 and a pharmaceutically acceptable carrier.

20. A pharmaceutical composition for the prevention or treatment of coronavirus infection in a subject, comprising the antibody or antigen-binding fragment thereof according to any one of claims 1-14.

21. Use of the antibody or antigen-binding fragment thereof according to any one of claims 1-14 in the production of a pharmaceutical composition for the prevention or treatment of coronavirus infection in a subject.

22. The pharmaceutical composition according to claim 19 or 20, wherein it is administered in combination with another anti-coronaviral drug.

23. The pharmaceutical composition according to claim 22, wherein the anti-coronaviral drug is at least one anti-coronaviral drug selected from remdesivir, favipiravir, dexamethasone, ciclenisonide, nafamostat, camostat, ivermectin, bamilanibimab, etesevimab, casilibimab, imdevimab, and HIV protease inhibitors.
